# EUROPEAN PATENT SPECIFICATION

(11) **EP 2 623 024 B1**
(45) Date of publication and mention of the grant of the patent: **16.09.2020**
(21) Application number: 11829039.4
(22) Date of filing: 26.09.2011
(51) Int. Cl.: A61B 5/0225, A61B 5/022

(54) **ELECTRONIC BLOOD PRESSURE METER AND METHOD FOR MEASURING BLOOD PRESSURE**
ELEKTRONISCHES BLUTDRUCKMESSGERÄT UND VERFAHREN ZUR BLUTDRUCKMESSUNG
TENSIOMÈTRE ARTÉRIEL ÉLECTRONIQUE ET PROCÉDÉ DE MESURE DE LA PRESSION SANGUINE

(30) Priority: 27.09.2010 JP 2010214789
(43) Date of publication of application: 07.08.2013
(73) Proprietor: Japan Precision Instruments Inc., Shibukawa-shi Gunma 377-0293 (JP)
(72) Inventor: TAKAHASHI, Yukio, Shibukawa-shi Gunma 377-0293 (JP)
(74) Representative: Betten & Resch
(86) International application number: PCT/JP2011/071905
(87) International publication number: WO 2012/043485

(56) References cited:
- WO-A1-2010/013554
- WO-A1-2010/089917
- JP-A- 63 186 625
- JP-A- 2005 304 670
- JP-A- 2009 279 196
- JP-A- 2009 297 161
- JP-B2- 3 925 858
- US-A1- 2004 127 801

## Description

### {Technical Field}

The present invention relates to an electronic sphygmomanometer for conducting non-invasive blood pressure measurement and a method of measuring blood pressure which are applied to the non-invasive blood pressure measurement.

### {Background Art}

Generally, non-invasive blood pressure measurement is conducted in such a way that an arm, wrist, or other location over an artery (hereafter, simply referred to as a "measurement site") in human subjects or animal subjects is wrapped by a cuff band. In this process, it is desirable that the cuff band wrapped around the measurement site have an appropriate width (which is the length in the direction perpendicular to the wrapping direction of the cuff band) to the circumferential length of the measurement site. The reason is that if a cuff band with an inappropriate width is used, the following well-known phenomena are likely to occur: if the cuff width is narrower than the optimal value, a blood pressure measurement result tends to be observed to be high; and if the cuff width is wider than the optimal value, a blood pressure measurement result tends to be observed to be low.

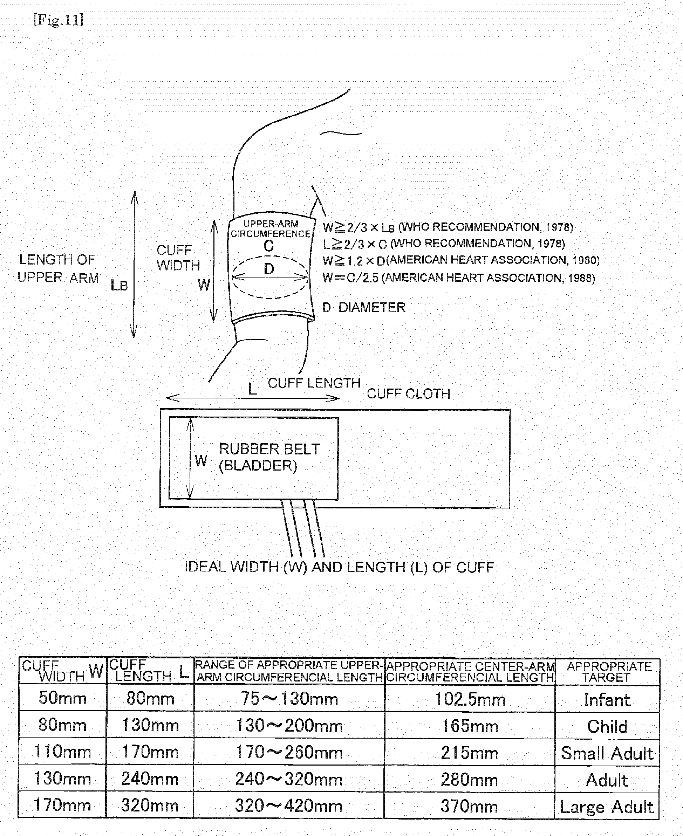

Although it is ideal to prepare a cuff band having an appropriate width to the circumferential length of each measurement site of every test subject, doing so is actually very difficult. Therefore, for example, as shown in FIG. 11, it is generally recommended that the circumferential length of the measurement site be divided into a plurality of ranges (refer to the "range of the adaptable upper-arm circumferential length" in the drawing), and a cuff band with a width (refer to the "Cuff width W" in the drawing) that is suitable for the substantially center circumferential length of each range (refer to the "adaptable center arm circumferential length" in the drawing) be used to measure the blood pressure of the test subject having an upper-arm circumferential length of the range. That is, the range of the circumferential length that allows a blood pressure measurement error in a cuff band with a certain "cuff width W" to be within an allowable range is defined as a "range of the adaptable upper-arm circumferential length" for the cuff band; and cuff bands with the plurality of "cuff widths W" are beforehand prepared so that combinations of a plurality of "ranges of the adaptable upper-arm circumferential length" can cover the expected range of the circumferential length of the measurement site of all test subjects.

However, as stated above, even if cuff bands with a plurality of sizes are prepared and either one is selectively used, strictly speaking, as the value of the circumferential length of the measurement site is deviated from the adaptable center arm circumferential length within the range of each adaptable upper-arm circumferential length, an error is probably generated in the measurement result of the blood pressure value. For this reason, attempts have recently been made to automatically correct the measurement error generated from the correlation between the circumferential length of the measurement site and the width of the cuff band.

Specifically, for example, it has been proposed that measurement of the discharge quantity of a pressure pump which pressurizes the inside of a gas bag included in the cuff band is conducted, and the circumferential length of the measurement site is calculated based on the correlation between the pressure in the gas bag and the measured discharge quantity, and the blood pressure measurement result is then corrected based on the calculated circumferential length of the measurement site (e.g., refer to patent literature 1).

Furthermore, with regard to the circumferential length of the measurement site, for example, it has been proposed that the circumferential length is detected by measuring a resistance value of a sliding resistor disposed in the cuff band, or the circumferential length is estimated based on the time required for pressurizing the cuff band to a specific pressure (e.g., refer to patent literature 2).

Document WO 2010/089917 A1 discloses a blood-pressure measuring device with a wrapping strength detecting unit for detecting the wrapping strength of a cuff around a measurement site. The wrapping strength detecting unit detects the wrapping strength of the cuff before the blood-pressure value is calculated by a blood-pressure calculating unit. More specifically, in the process of controlling the cuff pressure by a pressure regulating unit, the wrapping strength of the cuff is detected on the basis of the relationship indicated by the volumetric change of the cuff which is detected with the change of the detected pressure in the cuff wrapped around the measurement site from P1 to P2 and the volumetric change of the cuff which is detected with the change from P2 to P3.

### {Citation List}

### {Patent Literature}

Patent literature 1: Japanese Patent No. 3925858
Patent literature 2: Japanese Unexamined Patent Application Publication No. Hei 6(1994)-245911
**Patent literature 3:** WO 2010/089917 A1

### {Summary of Invention}

### {Technical Problem}

According to the conventional technology disclosed in patent literature 1, a measurement error generated from the correlation between the circumferential length of a measurement site and the width of a cuff band can be eliminated; however, it becomes necessary to measure the discharge quantity of the pressure pump in the process of pressurizing the inside of the gas bag. More specifically, for example, it becomes necessary to provide a pressure pump with an additional special detecting function to measure the discharge quantity of the pressure pump in such a way that the pressure pump is provided with a photo-interrupter, the number of rotations of the pressure pump in the specific pressure area during the pressurization process is detected and measured by the photo-interrupter, and then the discharge quantity of the pump is measured. Therefore, when compared with the general configuration in which the pressure pump does not require a special detecting function, the additional detecting function inevitably increases production costs as well as increasing the risk of the occurrence of a failure.

Furthermore, as disclosed in patent literature 2, when a circumferential length is detected by measuring a resistance value of a sliding resistor disposed in a cuff band, the structure of the cuff band becomes complicated due to the installment of the sliding resistor in the cuff band. Thus, it is presumed that such a configuration is likely to cause a failure and increase production costs.

Furthermore, as disclosed in patent literature 2, when the circumferential length is estimated based on the time necessary for pressurizing the cuff band to a specific pressure, it is presumed that the correct circumferential length may not be able to be estimated due to the occurrence of an error in an estimated result of the circumferential length because of uneven pressurizing capabilities of individual pressure pumps or fluctuation of pressurization time because of the voltage fluctuation of the pressure pump drive power source.

An objective of the present invention is to provide an electronic sphygmomanometer capable of accurately obtaining a circumferential length of a measurement site without providing a complicated mechanism for a cuff band or pressurizing means for the cuff band, and a method of measuring blood pressure.

### {Solution to Problem}

This problem is solved by an electronic sphygmomanometer according to claim 1 and by a method of measuring blood pressure according to claim 8. Advantageous embodiments are defined in the dependent claims.

The present invention is provided to achieve the above-mentioned object.

A first aspect provides an electronic sphygmomanometer including:
comprising:
a cuff band wrapped around a measurement site;
a fluid bag included in the cuff band;
pressurizing means for pressurizing an inside of the fluid bag by supplying a fluid into the fluid bag;
an electric depressurizing valve for reducing a pressure in the fluid bag by discharging the fluid from the fluid bag pressurized to a specific pressure;
an electric depressurizing valve drive circuit for regulating a drive energy amount of the electric depressurizing valve for controlling a discharge flow rate of the fluid discharged by the electric depressurizing valve, to thereby reduce a pressure in the fluid bag at a specific depressurizing speed; and
circumferential length estimating means for obtaining a circumferential length of the measurement site by using a drive energy amount regulated by the electric depressurizing valve drive circuit and previously set basic data.

A second aspect provides the electronic sphygmomanometer according to the first aspect, including:
blood pressure measuring means for conducting blood pressure measurement to the measurement site in a process of reducing a pressure in the fluid bag; and
measurement result correcting means for correcting a blood pressure measurement result obtained by the blood pressure measuring means based on the circumferential length of the measurement site obtained by the circumferential length estimating means.

A third aspect provides the electronic sphygmomanometer according to the first aspect or the second aspect, wherein the drive energy amount is a drive current value of the electric depressurizing valve.

A fourth aspect provides the electronic sphygmomanometer according to the third aspect, including:
a depressurizing controller for giving a regulating instruction to the electric depressurizing valve drive circuit, the regulating instruction being an instruction of regulating a drive current value by sending a control signal showing information equivalent to the drive current value of the electric depressurizing valve, wherein
the circumferential length estimating means obtains a circumferential length of the measurement site by using the control signal sent to the electric depressurizing valve drive circuit from the depressurizing controller.

A fifth aspect provides the electronic sphygmomanometer according to any one of the first to fourth aspects, wherein the basic data is configured to define a correlation between the pressure in the fluid bag and the drive energy amount of the electric depressurizing valve, so that the circumferential length of the measurement site corresponding to the drive energy amount or the information corresponding thereto can be specified when the pressure value in the fluid bag and the drive energy amount of the electric depressurizing valve or the information corresponding thereto are given.

A sixth aspect provides the electronic sphygmomanometer according to any one of the first to fifth aspects, wherein the circumferential length estimating means obtains circumferential lengths of measurement sites at a plurality of pressure values in a process of depressurizing an inside of the fluid bag, and specifies one reference circumferential length from the circumferential lengths at the plurality of pressure values.

A seventh aspect provides the electronic sphygmomanometer according to any one of the first to sixth aspects, including:
data storage means for storing the basic data; and connecting means for connecting an adjustment jig for conducting measurement for specifying the basic data and writing the basic data obtained by the measurement into the data storage means.

An eighth aspect provides a method of estimating a circumferential length of a measurement site, comprising:
pressurizing an inside of a fluid bag by supplying a fluid into the fluid bag while a cuff band internally including the fluid bag is wrapped around a measurement site;
reducing a pressure in the fluid bag by discharging the fluid from the fluid bag pressurized to a specific pressure by using an electric depressurizing valve, and regulating a drive energy amount of the electric depressurizing valve to control a discharge flow rate of the fluid discharged by the electric depressurizing valve so that a depressurizing speed becomes a specific speed when a pressure of the inside of the fluid bag is reduced; and
obtaining a circumferential length of the measurement site by using the drive energy amount of the electric depressurizing valve after regulation, and previously set basic data.

A ninth aspect provides a method of measuring blood pressure, comprising:
pressurizing an inside of the fluid bag by supplying a fluid into the fluid bag while a cuff band internally including the fluid bag is wrapped around a measurement site;
reducing a pressure in the fluid bag by discharging the fluid from the fluid bag pressurized to a specific pressure by using an electric depressurizing valve, and regulating a drive energy amount of the electric depressurizing valve to control a discharge flow rate of the fluid discharged by the electric depressurizing valve so that a depressurizing speed becomes a specific speed when a pressure of the inside of the fluid bag is reduced;
obtaining a circumferential length of the measurement site by using the drive energy amount of the electric depressurizing valve after regulation, and previously set basic data;
conducting blood pressure measurement to the measurement site in a process of reducing the pressure in the fluid bag; and
correcting a result of a blood pressure measurement applied to the measurement site based on the circumferential length of the measurement site obtained by using the drive energy amount of the electric depressurizing valve.

### {Advantageous Effects of Invention}

A circumferential length of the measurement site is obtained by use of a drive energy amount of the electric depressurizing valve; therefore, the circumferential length can be obtained by using a function originally possessed by an electronic sphygmomanometer, without adding a detecting function, etc., specific to the cuff band and the pressurizing means. Therefore, when compared with the configuration that requires a special detecting function, there is a less risk of the occurrence of a failure and production costs can be easily reduced. Furthermore, since the circumferential length of the measurement site is obtained in a process of reducing the pressure in the fluid bag, the circumferential length can be precisely obtained, if compared with a case that the circumferential length of the measurement site is obtained in the process of pressurizing an inside of the fluid bag.

### {Brief Description of Drawings}

FIG. 1 is an explanatory view showing an example of the schematic configuration of the electronic sphygmomanometer according to the present invention.
FIG. 2 is an explanatory view of an example of a using mode of the electronic sphygmomanometer according to the present invention.
FIG. 3 is a block diagram showing an example of functional configuration of the electronic sphygmomanometer according to the present invention.
FIG. 4 is an explanatory view showing a specific example of the Ragan & Bordley correction table.
FIG. 5 is an explanatory view showing a specific example of the correlation between the air volume in the bladder and the circumferential length of the measurement site.
FIG. 6 is an explanatory view showing a specific example of the correlation of the pressure in the bladder, circumferential length of the measurement site, and a drive current value of an electric depressurizing valve.
FIG. 7 is an explanatory view showing a specific example of the correlation of the pressure in the bladder, circumferential length of the measurement site, and a value of a D/A output signal.
FIG. 8 is a block diagram showing a configuration example of an electronic sphygmomanometer according to the present invention and an adjustment jig used by being connected to the electronic sphygmomanometer.
FIG. 9 is a flow chart showing a specific example of a pre-processing conducted by the electronic sphygmomanometer according to the present invention.
FIG. 10 is a flow chart showing an example of a specific procedure of the method of measuring a blood pressure by using the electronic sphygmomanometer according to the present invention.
FIG. 11 is an explanatory view showing an outline of a cuff width in a general cuff band.

### {Description of Embodiments}

Hereafter, an electronic sphygmomanometer according to the present invention, a method of estimating a circumferential length of the measurement site, and a method of measuring a blood pressure according to the present invention are described with reference to the drawings.

Herein, an outline of an electronic sphygmomanometer according to the present invention is first explained, and then a configuration example of the electronic sphygmomanometer is explained in detail. In the description of the configuration example, functional configuration of the electronic sphygmomanometer is first described, various parameters of the devices used for the electronic sphygmomanometer are then explained, and subsequently, the adjustment jig that can be connected to the electronic sphygmomanometer is described. After the configuration example has been described, a method of measuring blood pressure that uses the electronic sphygmomanometer is described to offer an example of the operation of the electronic sphygmomanometer. The method of estimating a circumferential length of the measurement site is assumed to be a part of the method of measuring blood pressure according to the present invention.

### <1. Outline of the electronic sphygmomanometer>

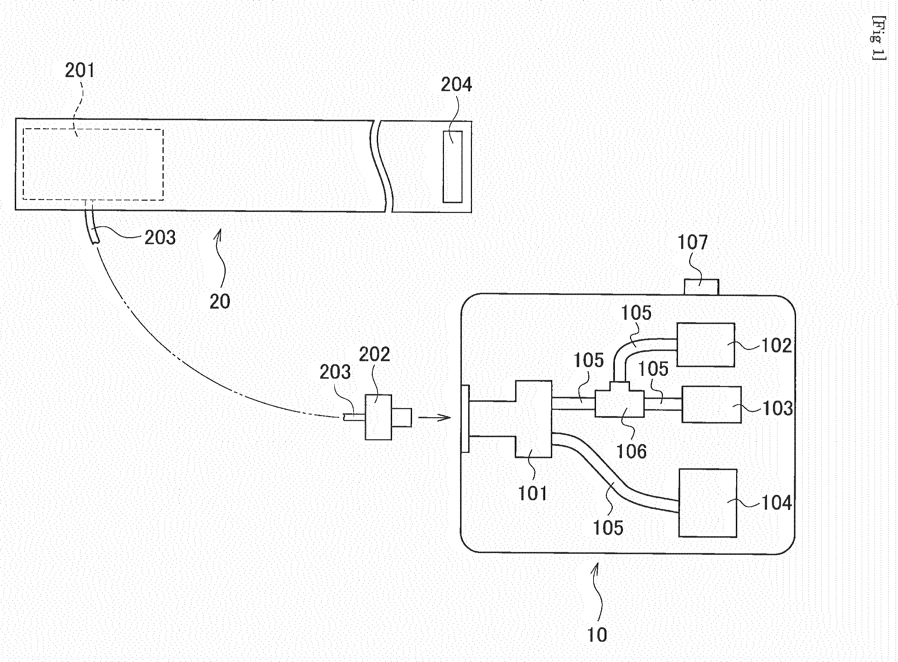

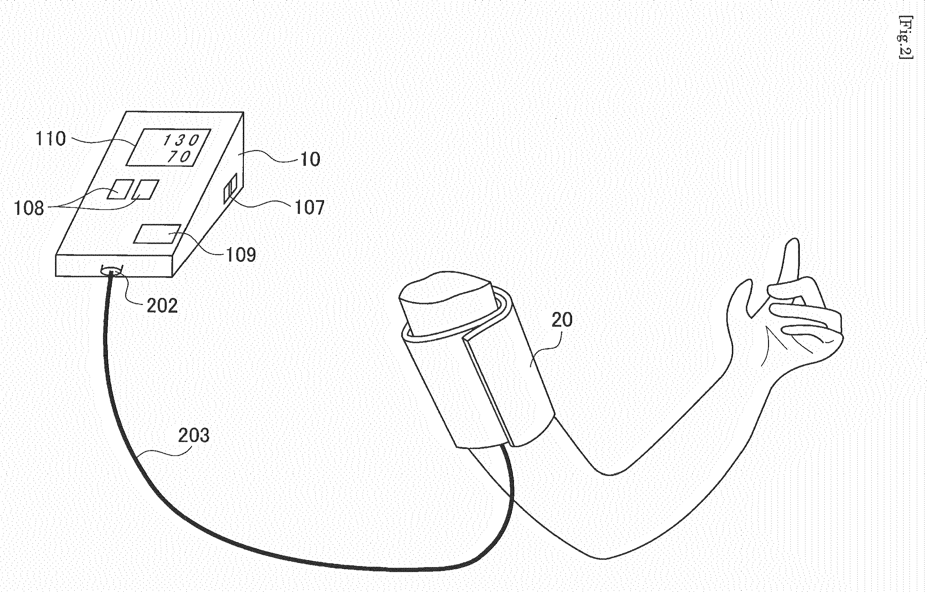

FIG. 1 is an explanatory view showing an example of the schematic configuration of the electronic sphygmomanometer according to the present invention. FIG. 2 is an explanatory view of an example of the use embodiment of the electronic sphygmomanometer according to the present invention.

The electronic sphygmomanometer, described below, is intended to conduct a non-invasive blood pressure measurement and, as shown in FIG. 1, includes a sphygmomanometer body 10 and a cuff band 20 wrapped around the measurement site of a human test subject or an animal test subject (hereafter, simply referred to as a "subject").

The cuff band 20 internally includes a fluid bag called a "bladder" 201. The bladder 201 is designed so that air is sent to the bladder 201 from the sphygmomanometer body 10 through a plug 202 and a tube 203. However, fluid other than air (specific gas, liquid, etc.) may also be sent to the bladder. Furthermore, the cuff band 20 is provided with a hook-and-loop fastener 204 so that the cuff band 20 remain secure after being wrapped around the measurement site. Although the cuff band 20 and the bladder 201 are generally made of different materials, they can be integrally formed by applying treatment such as welding to a single material.

Meanwhile, the sphygmomanometer body 10 includes a connector 101 into which a plug 202 is inserted, a pressure pump 102 for sending air into the bladder 201, and an electric depressurizing valve 103 for regulating low depressurizing speed (slow leak speed) in the bladder 201 in the blood pressure measurement process. Furthermore, the sphygmomanometer body 10 includes a pressure sensor 104 for measuring the pressure in the bladder 201, a tube 105 for connecting the above-mentioned devices, and a joint 106. Also, the sphygmomanometer body 10 includes a power switch 107 on the outer surface thereof.

Furthermore, as shown in FIG. 2, the sphygmomanometer body 10 includes an operation button 108, a start button 109, and an LCD (Liquid Crystal Display) 110 on the outer surface thereof.

When measuring a blood pressure by use of an electronic sphygmomanometer thus configured, the power switch 107 of the sphygmomanometer body 10 is first turned on. Then, as shown in FIG. 2, the cuff band 20 is wrapped around the measurement site of a subject (e.g., an upper arm of the subject). Next, the operation button 108 is operated, and a pressure value corresponding to an approximate highest blood pressure value of a subject is selected as a pressurization target value applied to the bladder 201. Subsequently, when the start button 109 is pressed, air is sent from the sphygmomanometer body 10 to the bladder 201 through the tube 203 and measurement of the blood pressure of the subject begins. Specifically, the inside of the bladder 201 is pressurized to a target pressure value to thereby press the measurement site of the subject, etc., so that a blood flow to a peripheral site is temporarily stopped. After that, the electric depressurizing valve 103 is operated to discharge air from the bladder 201, and the pressure in the bladder 201 is gradually reduced at a low depressurizing speed. The blood pressure value of the subject, etc., is measured by observing a pulsation change and appearance of Korotkoff sounds in the artery in the process of gradually reducing a pressure added on the measurement site. Moreover, the LCD 110 disposed on the sphygmomanometer body 10 displays the change of pressure in the bladder 201 during the measurement as well as the systolic blood pressure value and the diastolic blood pressure value which are the measurement results. After the blood pressure measurement is completed, a pulse rate may also be displayed.

### <2. Detailed description of the configuration example of the electronic sphygmomanometer>

Next, the configuration example of the electronic sphygmomanometer is further explained in detail. Functional configuration of the electronic sphygmomanometer is first explained, various device parameters used for the electronic sphygmomanometer are then explained, and subsequently, the adjustment jig that can be connected to the electronic sphygmomanometer is described.

### (2-1. Functional configuration of the electronic sphygmomanometer)

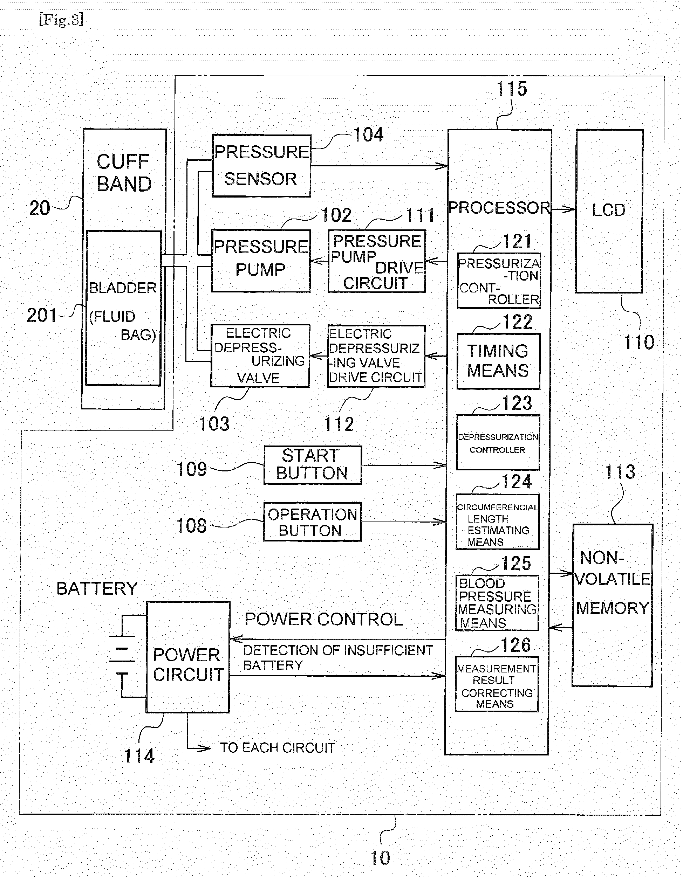

FIG. 3 is a block diagram showing an example of a functional configuration of the electronic sphygmomanometer according to the present invention.

As shown in the example of the figure, the electronic sphygmomanometer includes a pressure pump drive circuit 111, an electric depressurizing valve drive circuit 112, a nonvolatile memory 113, a power circuit 114, and a processor 115 in addition to the above-mentioned cuff band 20 that internally includes the bladder 201, pressure pump 102, electric depressurizing valve 103, pressure sensor 104, operation button 108, start button 109, and the LCD 110. Those configuration elements other than the cuff band 20 are disposed in the sphygmomanometer body 10.

The pressure pump drive circuit 111 applies a drive current to the pressure pump 102. As the result of the application of the drive current by the pressure pump drive circuit 111, the pressure pump 102 supplies air into the bladder 201, thereby pressurizing an inside of the bladder 201. That is, the pressure pump 102 and the pressure pump drive circuit 111 function as pressurizing means for pressurizing the inside of the bladder 201. However, the pressurizing means is not limited to the pressure pump 102 and the pressure pump drive circuit 111, provided that the inside of the bladder 201 is pressurized; that is, for example, a manual pump may be used as the pressurizing means.

The electric depressurizing valve drive circuit 112 applies drive current to the electric depressurizing valve 103 and also regulates the drive current value. Here, the drive current value is equivalent to a specific example of the drive energy amount of the electric depressurizing valve 103 (physical amount for controlling the drive of the electric depressurizing valve 103). By applying and adjusting the drive current which is a specific example of the drive energy amount, the electric depressurizing valve 103 discharges air from the bladder 201 pressurized to a specific pressure (the pressurization target value for the subject) to reduce the pressure in the bladder 201 and also controls the discharge flow rate of the air discharged by the electric depressurizing valve 103 so that the depressurizing speed (slow leak speed) becomes a specific speed. That is, the electric depressurizing valve 103, illustrated herein, is so configured that the valve goes into a fully closed state when a specific drive energy amount is applied, thereby completely preventing deflation; and with the gradual reduction of the drive energy amount, the degree of valve opening increases, thereby increasing the discharge flow rate at which air is discharged; and when the drive energy amount is further reduced to a specific value or less, the valve goes into a fully open state, thereby rapidly discharging air.

Furthermore, the electric depressurizing valve drive circuit 112 includes a constant current circuit. The constant current circuit is a circuit in which stable current flows without being affected by fluctuations of power-supply voltage or temperature.

Specifically, the electric depressurizing valve drive circuit 112 is configured to control the electric depressurizing valve 103 by flowing a constant current proportional to the D/A output signal value, with its resolving power of 12 bits, based on a D/A output signal outputted from a D/A port of a processor 115 described later.

The nonvolatile memory 113, including an EEPROM (Electrically Erasable and Programmable Read Only Memory) for example, stores and holds various data. The various data includes basic data described later in detail. Namely, the nonvolatile memory 113 functions as data storage means for storing basic data.

The power circuit 114 supplies power to each circuit (pressure pump drive circuit 111, electric depressurizing valve drive circuit 112, and processor 115, etc.) in the sphygmomanometer body 10. It can be considered that input power to the power circuit 114 is supplied from a primary battery or a secondary battery disposed in the sphygmomanometer body 10. However, the input power from a commercial power source, namely the input power supplied from outside of the sphygmomanometer body 10 is also acceptable.

The processor 115 including a combination of a CPU (Central Processing Unit) and RAM (Random Access Memory) controls the overall operation of the electronic sphygmomanometer based on a prescribed program. More specifically, by executing the prescribed program by the CPU, the processor 115 functions as a pressurizing controller 121, a timing means 122, a depressurizing controller 123, a circumferential length estimating means 124, a blood pressure measuring means 125, or a measurement result correcting means 126.

When the start button 109 is pressed, the pressurizing controller 121 sends an instruction to the pressure pump drive circuit 111 so that the pressure pump 102 pressurize the inside of the bladder 201 to the pressurization target value.

When the electric depressurizing valve 103 starts to depressurize in the bladder 201, the timing means 122 measures the elapsed time from starting the depressurization.

When the pressure in the bladder 201 detected by the pressure sensor 104 becomes a specific pressure (the pressurization target value for the subject), the depressurizing controller 123 sends an instruction to the electric depressurizing valve drive circuit 112 so that the electric depressurizing valve 103 depressurizes the inside of the bladder 201. However, the depressurizing controller 123 controls depressurizing operation conducted by the electric depressurizing valve 103 by feedback so that the pressure in the bladder 201 is depressurized at a specific speed. Specifically, based on the pressure result detected by the pressure sensor 104 and the timing result obtained by the timing means 122, the drive current value of the electric depressurizing valve 103 is selected, namely the drive current value of the electric depressurizing valve 103 regulated by the electric depressurizing valve drive circuit 112 is selected, so that the depressurizing by the electric depressurizing valve 103 is performed at a preset constant speed. That is, the drive current value of the electric depressurizing valve 103 regulated by the electric depressurizing valve drive circuit 112 is selected.

When the drive current value is selected, an instruction of regulating the drive current is given to the electric depressurizing valve drive circuit 112 by giving information equivalent to the selected drive current value, to the electric depressurizing valve drive circuit 112. Herein, the information "equivalent to" the drive current value means the information that works in the same manner as the drive current value, corresponding to a control signal for example, given to the electric depressurizing valve drive circuit 112 to thereby indicate the drive current value. An example of a control signal is a voltage output signal obtained by converting digital data on the processor 115 to analog data. Hereafter, the voltage output signal, which is an example of the control signal, is referred to as a "D/A output signal". That is, the depressurizing controller 123 instructs the electric depressurizing valve drive circuit 112 to regulate the drive current by sending the D/A output signal.

The circumferential length estimating means 124 obtains a circumferential length of the measurement site wrapped by the cuff band 20 in the process of reducing the pressure in the bladder 201 by the electric depressurizing valve 103. More specifically, at the time of the depressurization in the bladder 201, the circumferential length estimating means 124 uses the drive current of the electric depressurizing valve 103 regulated by the electric depressurizing valve drive circuit 112 to obtain the circumferential length of the measurement site. Herein, the "use" of the drive current means the use of the drive current so as to be useful for obtaining the circumferential length of the measurement site. Consequently, as far as the drive energy amount of the electric depressurizing valve 103 is used, the electric current value itself is not necessarily required to be used (e.g., the amount of power). For this reason, the circumferential length estimating means 124 specifically uses the D/A output signal that is an example of the information equivalent to the drive current value, namely the D/A output signal given to the electric depressurizing valve drive circuit 112 by the depressurizing controller 123, to obtain the circumferential length of the measurement site.

Furthermore, the circumferential length estimating means 124 obtains the circumferential length of the measurement site in such a way that the circumferential length corresponding to each of a plurality of pressure values is obtained in the process of depressurizing the inside of the bladder 201, and one reference circumferential length is specified from each circumferential length corresponding to each of the plurality of pressure values. Herein, the one reference circumferential length specified from each circumferential length can be, for example, a mean value of each circumferential length; however, it may be a most frequently-appearing value or an intermediate value (value located between the maximum value and the minimum value).

As described in detail later, the circumferential length estimating means 124 obtains the circumferential length of the measurement site while referring to basic data previously set and stored in the nonvolatile memory 113.

The blood pressure measuring means 125 performs blood pressure measurement to the measurement site wrapped by the cuff band 20 in the process of depressurizing the inside of the bladder 201. Blood pressure measurement may be conducted by means of well-known methods. For example, it can be considered that based on the result detected by the pressure sensor 104 in the process of gradually reducing the pressure added on the measurement site, pulse components that are superposed on the detected pressure data are sorted, and that a blood pressure value is determined by Oscillometric method, from the sorted pulse signal and pressure data. It can be further considered that the blood pressure value is determined according to Korotkoff sounds generated in the process of gradually reducing the pressure added on the measurement site. Moreover, for example, the blood pressure value may be determined by using the oscillometric method and the Korotkoff sound method concurrently.

The measurement result correcting means 126 has a function of correcting the blood pressure measurement result obtained by the blood pressure measuring means 125 based on the circumferential length of the measurement site obtained by the circumferential length estimating means 124. When the circumferential length estimating means 124 specifies one reference circumferential length, the blood pressure measurement result may be corrected based on the reference circumferential length. It can be considered that the Ragan & Bordley correction table or the Pickering correction formula, for example, is applied to correct the blood pressure measurement result.

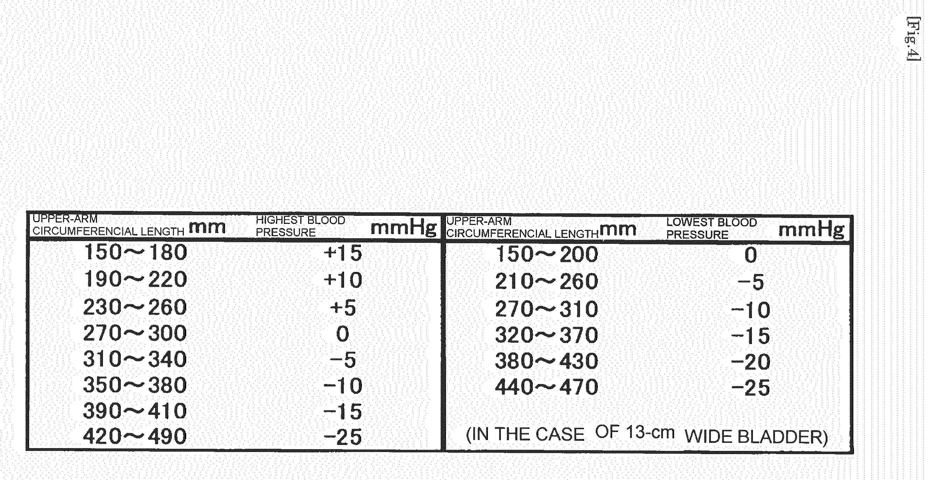

FIG. 4 is an explanatory view showing a specific example of the Ragan & Bordley correction table. The table in the view indicates that when a 13-cm wide bladder 201 is used, the systolic blood pressure value and the diastolic blood pressure value are changed according to the circumferential length of the upper arm. For example, the blood pressure measurement result is corrected for the subject having an upper arm circumferential length of 240mm, in such a way that 5mmHg is added to a measured highest blood pressure value, and 5mmHg is subtracted from a measured lowest blood pressure value.

The Pickering correction formula is as follows: s:d = 1.27c - 35.86:0.87c - 15.54, where s: highest blood pressure value (mmHg), c: upper-arm circumferential length (mm), and d: lowest blood pressure value (mmHg).

Correcting the blood pressure measurement result is not limited thereto, and other well-known methods may also be used.

### (2-2. Device parameters in the electronic sphygmomanometer)

Next, various device parameters used for the electronic sphygmomanometer and the correlation of the device parameters will be explained with reference to a specific example.

The "device parameter", described herein, is a variable numerical value or index in the electronic sphygmomanometer when measuring the blood pressure by using an electronic sphygmomanometer. Specifically, the air volume in the bladder 201, circumferential length of the measurement site wrapped by the cuff band 20 which internally includes the bladder 201, pressure in the bladder 201, the drive current value of the electric depressurizing valve 103 used for depressurizing the inside of the bladder 201, and the D/A output signal which is an example of the information equivalent to the drive current value, correspond to the "device parameters" called here.

Regarding the cuff band 20, cuff bands having a plurality of cuff widths (having a size in a direction vertical to a wrapping direction of the cuff band 20) are generally prepared so as to cover the range of the circumferential length of the measurement site of all, etc. Various formation materials can be used to make cuff bands 20 and bladders 201.

Here, the cuff band 20 including a vinyl-chloride bladder 201, made of a nylon-cloth, having a largest ratio of an arm circumferential length distribution, called an "adult cuff", and having a size of 240mm to 320mm in the adaptable upper arm circumferential length range, is given as an example, for the following explanation.

Inventors of the present invention examine the correlation of various device parameters used for the electronic sphygmomanometer including the adult cuff. As a result, it is found that the device parameters have the correlation described below.

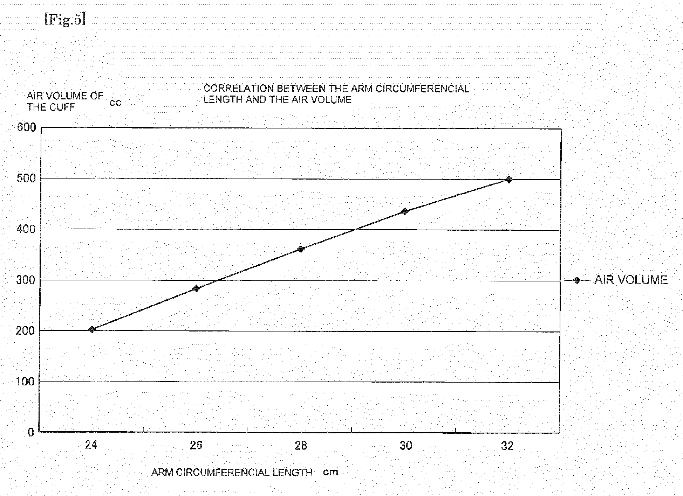

FIG. 5 is an explanatory view showing a specific example of the correlation between an air volume in the bladder and the circumferential length of the measurement site.

An air volume in the bladder 201 was measured when a cuff band 20 called an "adult cuff" was wrapped around an arm having a circumferential length of 240 mm which is a lower limit of the range of the adaptable upper-arm circumferential length, and the air volume in the bladder 201 was also measured when the cuff band 20 was wrapped around another arm having a circumferential length of 320 mm which is an upper limit of the range of the adaptable upper-arm circumferential length. Then, it is found by an actual measurement that as shown in FIG. 5, there is a correlation between the air volume and the circumferential length. According to the correlation shown in the figure, the air volume is increased or decreased approximately in proportion to the arm circumferential length. Namely, since the cuff width is constant within the same range of the adaptable upper-arm circumferential length, the circumferential length of the measurement site and the effective air volume in the bladder 201 have a unique proportional relationship.

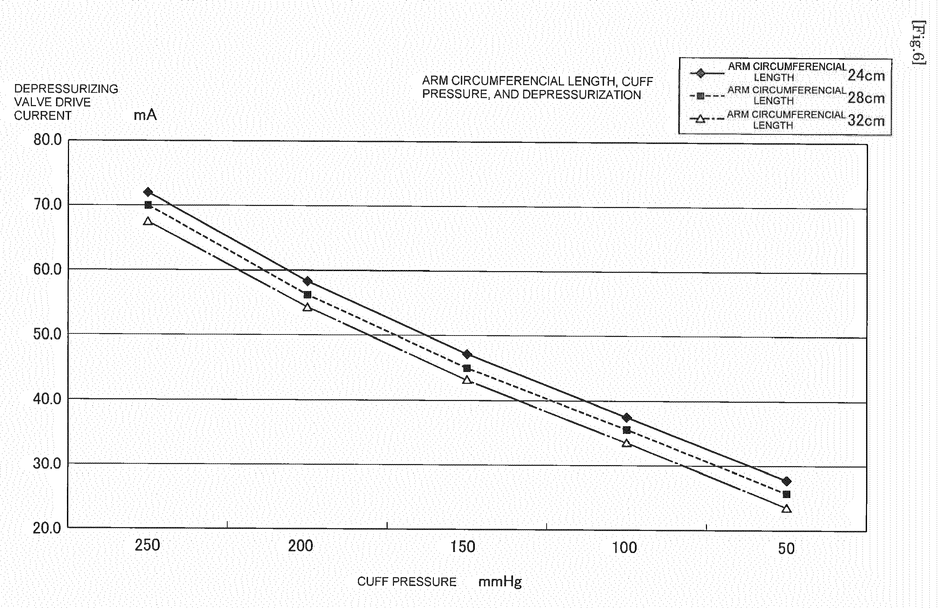

FIG. 6 is an explanatory view showing a specific example of the correlation of the pressure in the bladder, circumferential length of the measurement site, and the drive current value of the electric depressurizing valve.

When depressurizing the inside of the bladder 201, the depressurizing controller 123 performs feedback control regarding a depressurizing speed. In this feedback control, it is confirmed by the actual measurement that the correlation of FIG. 6 is obtained as the correlation of the bladder inner pressure, the measurement site circumferential length, and the electric depressurizing valve drive current value, when a target slow depressurizing speed is set to 3mm Hg/second for example.

According to the correlation shown in the figure, it is found that by such a feedback control to set the depressurizing speed to a constant speed, the drive current of the electric depressurizing valve 103 that performs depressurization, is varied corresponding to the pressure in the bladder 201 under control, provided that air volumes in the bladder 201 are same. Specifically, control is performed so as to maintain the depressurizing target speed by decreasing the discharge flow rate of air discharged from the bladder 201 (by increasing the degree of closing of the electric depressurizing valve 103) when the pressure in the bladder 201 is high, and by increasing the discharge flow rate of air discharged from the bladder 201 (by decreasing the degree of closing of the electric depressurizing valve 103) when the pressure in the bladder 201 is low.

Furthermore, according to the correlation shown in the figure, when the same pressure point in the depressurizing process is taken into consideration under the feedback control to set the depressurizing speed to a constant speed, it is found that a large air discharge flow rate is required from the bladder 201 during depressurization if the air volume in the bladder 201 is large (small closure of the electric depressurizing valve 103) so that the drive current of the electric depressurizing valve 103 can be small, and meanwhile, a large drive current is required for obtaining a small air discharge flow rate from the bladder 201 during depressurization (large closure of the electric depressurizing valve 103) if the air value from the bladder 201 is small.

The above-mentioned description is in accordance with the characteristics of the electric depressurizing valve 103 used in this embodiment. If an electric depressurizing valve having different characteristics is used, it is obvious that control different from the above-mentioned control be conducted.

The correlations shown in FIG. 5 and FIG. 6 are focused by the inventors of the present invention, and it is found that from the drive current value of the electric depressurizing valve 103, the circumferential length of the measurement site corresponding to this drive current value can be derived. Specifically, it is found that the drive current of the electric depressurizing valve 103 is regulated under the feedback control of the depressurizing speed, wherein the correlations shown in FIG. 5 and FIG. 6 are focused, and based on these correlations, the drive current of the electric depressurizing valve 103 after regulation is utilized, to thereby obtain the circumferential length of the measurement site.

Incidentally, the DC resistance value of the electric depressurizing valve 103 is constant. Therefore, the value of the D/A output signal given to the electric depressurizing valve drive circuit 112 by the depressurizing controller 123 to control the drive of the electric depressurizing valve 103 has a proportional relationship with the drive current value of the electric depressurizing valve 103.

That is, there is a unique correlation between the value of the D/A output signal sent from the depressurizing controller 123 and the drive current value of the electric depressurizing valve 103.

Accordingly, the correlation illustrated in FIG. 6 applies not only to the drive current value of the electric depressurizing valve 103 but also to the D/A output signal value that uniquely corresponds to the drive current value.

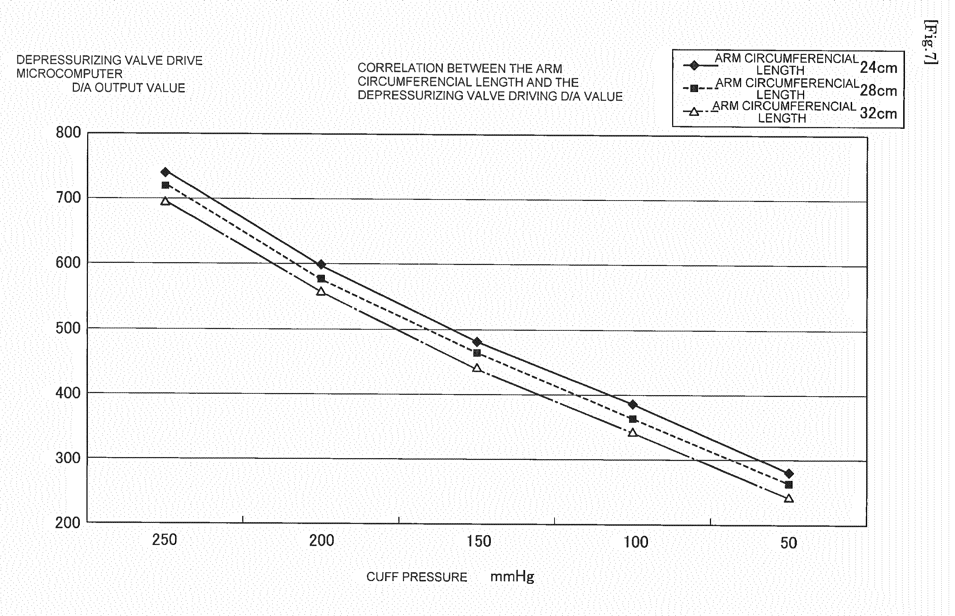

FIG. 7 is an explanatory diagram showing a specific example of the correlation of the pressure in the bladder, circumferential length of the measurement site, and the D/A output signal value.

According to the relation shown in the figure, the same pressure point in the depressurizing process is taken into consideration when the feedback control is performed so that the depressurizing speed is a constant speed. Then, it is found that if the air volume in the bladder 201 is large, a large D/A output signal value is not required, and meanwhile if the air volume in the bladder 201 is small, a large D/A output signal value is required.

Since there is a correlation shown in FIG. 7, it is found by the inventors of the present invention, that the circumferential length of the measurement site can be derived from the D/A output signal given to the electric depressurizing valve drive circuit 112 by the depressurizing controller 123 in the same manner as the above-mentioned drive current of the electric depressurizing valve 103. Namely, it is found that the correlations shown in FIG. 5 and FIG. 7 is focused, and based on these relations, the circumferential length of the measurement site can be obtained using the D/A output signal sent to the electric depressurizing valve drive circuit 112 from the depressurizing controller 123.

Regarding the correlation shown in FIG. 6 or FIG. 7, it is considered that typical one of the data for specifying the above-mentioned correlation, is previously set as basic data, and the previously set basic data is stored and held by the nonvolatile memory 113.

When an adult cuff is used, the basic data includes, for example, the data that defines the following correlation: the correlation between the pressure in the bladder 201 and the drive current of the electric depressurizing valve 103 when the cuff band 20 is wrapped around an upper arm with a circumferential length of 240 mm which is the lower limit of the range of the adaptable upper-arm circumferential length, and the correlation between the pressure in the bladder 201 and the drive current of the electric depressurizing valve 103 when the cuff band 20 is wrapped around an upper arm with a circumferential length of 320 mm which is the upper limit of the range of the adaptable upper-arm circumferential length. More specifically, for example, the air volume in the bladder 201 when the cuff band 20 is wrapped around an upper arm with a circumferential length of 240 mm and the air volume in the bladder 201 when the cuff band 20 is wrapped around an upper arm with a circumferential length of 320 mm are measured; and for each air volume, the drive current values of the electric depressurizing valve 103 at specific pressure points (e.g., every 20 mmHg from 260 mmHg to 40 mmHg) in the process of depressurizing the inside of the bladder 201 are measured; and the obtained air volume values, pressure values, and drive current values or the corresponding D/A output signal values are correlated. Then, the correlated data can be used as basic data.

Thus, basic data defines typical example of the correlation between device parameters used for the electronic sphygmomanometer. More specifically, by thus defining the correlations, the basic data is configured to specify the circumferential length of the measurement site corresponding to the drive current value or the D/A output signal value, when the pressure value in the bladder 201 and the drive current value of the electric depressurizing valve 103 or the D/A output signal value corresponding thereto are given.

When a plurality of cuff bands 20 with different cuff widths are prepared, basic data is expected to be previously set for each cuff band 20.

As described above, since the D/A output signal value uniquely corresponds to the drive current value, at least one of the data regarding the drive current value (see FIG. 6) and the data regarding the D/A output signal value (see FIG. 7) can be used as basic data. Hereafter, explanation is given for a case that the basic data regarding the D/A output signal value is given.

### (2-3. Configuration of the adjustment jig)

Next, an adjustment jig that used by being connected to the electronic sphygmomanometer will be described.

The adjustment jig is used by being connected to the electronic sphygmomanometer so that the electronic sphygmomanometer can conduct measurement to specify basic data and write the obtained basic data into the nonvolatile memory 113.

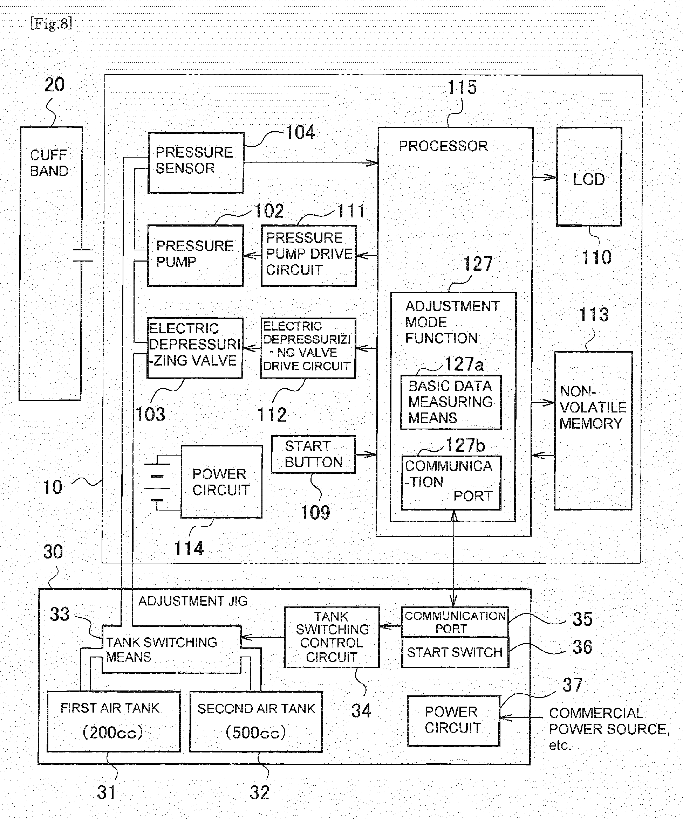

FIG. 8 is a block diagram showing a configuration example of an electronic sphygmomanometer according to the present invention and an adjustment jig used by being connected to the electronic sphygmomanometer.

To connect the adjustment jig 30, the processor 115 of the sphygmomanometer body 10 is provided with an adjustment mode function 127 in addition to the above-mentioned functions 121 to 126. The adjustment mode function 127 includes basic data measuring means 127a and a communication port 127b.

By the execution of a prescribed program by the CPU of the processor 115, the basic data measuring means 127a conducts measurement to specify basic data to be referred to by the circumferential length estimating means 124 and writes the obtained basic data into the nonvolatile memory 113.

The communication port 127b is, for example, a communication interface which enables bidirectional communication, such as a parallel port; and, the adjustment jig 30 can be connected via a communication cable. That is, the communication port 127b functions as connecting means for connecting the adjustment jig 30.

Meanwhile, the adjustment jig 30 used by being connected to the communication port 127b includes a first air tank 31, a second air tank 32, a tank switching means 33, a tank switching control circuit 34, a communication port 35, a start switch 36, and a power circuit 37.

The first air tank 31 and the second air tank 32 are sealed containers capable of storing different volumes of air, and each of them corresponds to their respective air volumes in the bladder included in the cuff band 20. When the adjustment jig 30 is taken as an example of the adult cuff, the first air tank 31 has an air volume of 200 cc, and the second air tank 32 has an air volume of 500 cc. This is because the air volume in the bladder 201 is approximately 200 cc when the cuff band 20 is wrapped around an upper arm having the arm circumferential length of 240 mm which is the lower limit of the range of the adaptable upper-arm circumferential length for the adult cuff, and the air volume in the bladder 201 is approximately 500 cc when the cuff band 20 is wrapped around an upper arm having the arm circumferential length of 320 mm which is the upper limit of the range of the adaptable upper-arm circumferential length for the adult cuff (e.g., see FIG. 4).

Thus, the adjustment jig 30 has a first air tank 31 and a second air tank 32 (i.e., two air tanks with different air volumes) per cuff band 20. However, the number of tanks is not limited to two, and the number of tanks may be three or more.

Furthermore, as long as the first air tank 31 and the second air tank 32 have different air volumes, they do not necessarily have to correspond to the lower limit or upper limit of the range of the adaptable upper-arm circumferential length. Even if those tanks do not correspond to the lower limit or upper limit, as long as their respective air volumes are different, calculation of the circumferential length of the measurement site is possible by means of the linear interpolation as will be described later.

The tank switching means 33 includes, for example, an airflow path switching mechanism, such as an electromagnetic valve, and is configured to switch the airflow paths to the first air tank 31 and the second air tank 32. More specifically, when the plug (not shown) of the adjustment jig 30, not the plug 202 of the cuff 20, is connected to the connector 101 of the sphygmomanometer body 10, the connection destination of the pressure pump 102, electric depressurizing valve 103, and the pressure sensor 104 included in the sphygmomanometer body 10 which is connected to the connector 101 is switched to either the first air tank 31 or the second air tank 32 located in the adjustment jig 30.

The tank switching control circuit 34 includes, for example, a controller of the electromagnetic valve and is configured to control the switching operation of the tank switching means 33. The switching operation can be controlled by sending a predetermined control signal from the tank switching control circuit 34 to the tank switching means 33.

The communication port 35 is provided for the connection to the communication port 127b of the sphygmomanometer body 10. When an electrical connection to the communication port 127b of the sphygmomanometer body 10 is ensured by means of the communication port 35, the adjustment jig 30 can bidirectionally send and receive various signals to and from the sphygmomanometer body 10.

The start switch 36 is operated by the user of the adjustment jig 30 (adjustment operator).

The power circuit 37 supplies power to the configuration elements (tank switching means 33, tank switching control circuit 34, etc.) of the adjustment jig 30. As the input power to the power circuit 37, a commercial power source supplied from the outside of the adjustment jig 30 can be used; however, a primary battery or secondary battery may be provided in the adjustment jig 30 to supply power.

### <3. Procedures for the method of measuring blood pressure>

Next, an example of the operation of the electronic sphygmomanometer, namely the specific procedures for the method of measuring a blood pressure by using the electronic sphygmomanometer, will be described. Herein, pre-processing necessary for conducting blood pressure measurement will be first explained, and the method of measuring a blood pressure will then be specifically described.

### (3-1. Pre-processing procedures)

The "pre-processing", described herein, refers to the measurement processing to determine basic data necessary for a blood pressure measurement and the processing of writing the obtained basic data into the nonvolatile memory 113. The pre-processing may be conducted prior to the blood pressure measurement of a subject, at the latest. Specifically, the pre-processing is expected to be conducted in the adjustment process during the production of the electronic sphygmomanometers (the last process before shipment).

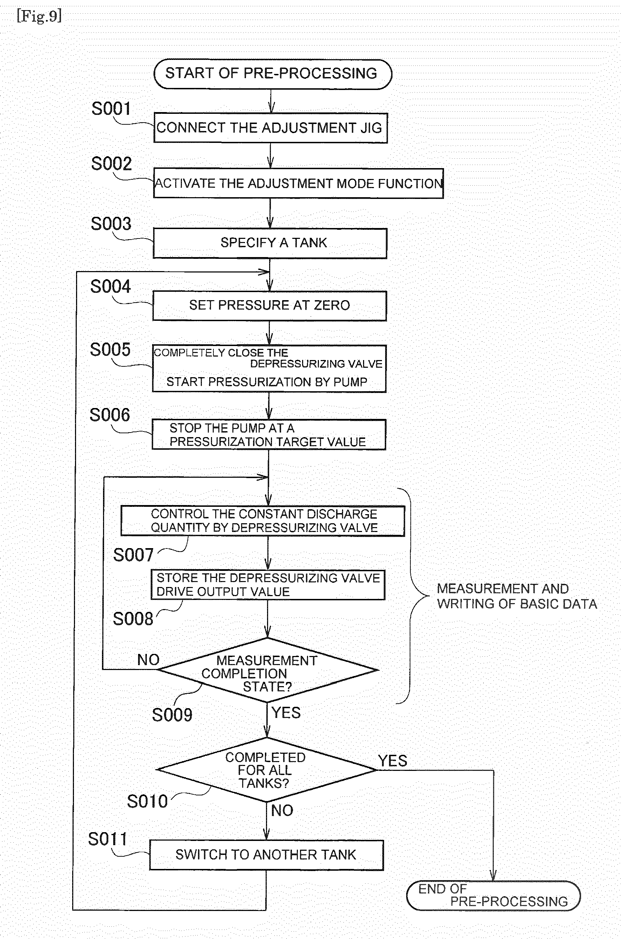

FIG. 9 is a flow chart showing a specific example of the pre-processing conducted by the electronic sphygmomanometer according to the present invention.

In performing the pre-processing, the adjustment jig 30 is prepared. Then, the adjustment operator connects the adjustment jig 30 to the sphygmomanometer body 10 (step 001; hereafter, the step is abbreviated as "S"). More specifically, the communication port 127b of the sphygmomanometer body 10 is connected to the communication port 35 of the adjustment jig 30 via a communication cable, and the plug (not shown) of the adjustment jig 30 is inserted into the connector 101 of the sphygmomanometer body 10.

After the sphygmomanometer body 10 is connected to the adjustment jig 30, the adjustment operator conducts predetermined operations using the power switch 107, start button 109, and the like located on the sphygmomanometer body 10. Thus, an adjustment mode function 127 is activated in the processor 115 of the sphygmomanometer body 10 (S002). However, it is also acceptable to activate the adjustment mode function 127 by an external control using the adjustment jig 30 through the communication ports 127b and 35. Specifically, for example, when the adjustment operator operates the start switch 36, the adjustment jig 30 sends a predetermined signal for switching an operation mode to an adjustment mode, to the sphygmomanometer body 10, through the communication ports 127b and 35. Thus, it is also acceptable to activate the adjustment mode function 127 in the processor 115 of the sphygmomanometer body 10.

When the adjustment mode function 127 is activated, the information for specifying the air tank connected to the sphygmomanometer body 10 is sent to the adjustment jig 30 from the sphygmomanometer body 10 by the basic data measuring means 127a through the communication ports 127b and 35 (S003). When receiving the information, in the adjustment jig 30, the tank switching control circuit 34 sends operating instructions to the tank switching means 33. As a result, in the adjustment jig 30, either the first air tank 31 or the second air tank 32 is connected to the sphygmomanometer body 10 as the air tank having the air volume specified by the sphygmomanometer body 10.

Herein, the example uses the case in which the second air tank 32 having an air volume of 500 cc, which corresponds to the upper limit of the range of the adaptable upper-arm circumferential length for an adult cuff, is connected to the sphygmomanometer body 10. After the second air tank 32 is connected, the relevant switching information is sent from the tank switching control circuit 34 to the sphygmomanometer body 10 through the communication ports 127b and 35.

When the switching information is received, in the sphygmomanometer body 10, the basic data measuring means 127a starts the basic data measurement. Specifically, while the sphygmomanometer body 10 is controlled by the basic data measuring means 127a, the pressure to be detected is automatically set at zero (reference alignment) by the pressure sensor 104 (S004); the electric depressurizing valve 103 is closed; the pressure pump 102 is activated; and the pressure in the second air tank 32 in the adjustment jig 30 begins to increase (S005). When the pressure in the tank is increased to a specific value (e.g., 280 mmHg), the drive of pressure pump 102 is stopped (S006). As long as the specific value is set at a value larger than the pressurization target value that can be set at the time of the blood pressure measurement, the specific value is not particularly limited.

After the pump is stopped, while the sphygmomanometer body 10 is still being controlled by the basic data measuring means 127a, the electric depressurizing valve 103 is gradually opened, and the pressure in the second air tank 32 in the adjustment jig 30 is reduced. At this point, the depressurizing controller 123 in the processor 115 of the sphygmomanometer body 10 performs control so as to conduct pressure reduction at a specific speed (e.g., 3 mmHg per second) based on the amount of pressure decrease per specific time (S007). Specifically, the depressurizing controller 123 sends instructions to the electric depressurizing valve drive circuit 112 so that, for example, when depressurizing speed is faster than 3 mmHg per second which is the depressurizing target speed, the drive current of the electric depressurizing valve 103 is increased; and when depressurizing speed is slower than 3 mmHg per second, the drive current is decreased. By means of such controls, normally in the period between the pressurization point (e.g., 280 mmHg) and a point at which pressure decreases by approximately 20 mmHg, the depressurizing speed becomes the pressure reduction target speed and then is stabilized.

After the depressurizing speed is stabilized, the sphygmomanometer body 10 recognizes the D/A output signal value corresponding to the drive current value of the electric depressurizing valve 103 at specific pressure points (e.g., every 20 mmHg from 260 mmHg to 40 mmHg) in the process of reducing the pressure in the second air tank 32 and writes the results into a predetermined storage area in the nonvolatile memory 113 (S008). Specifically, the value of the D/A output signal given to the electric depressurizing valve drive circuit 112 by the depressurizing controller 123, for example, at the pressure of 260 mmHg is first stored in the nonvolatile memory 113; and subsequently, the D/A output signal value at each pressure point is stored in the nonvolatile memory 113 every time the pressure decreases by 20 mmHg. After the D/A output signal value, for example, at the pressure of 40 mmHg, which is the lower limit of the measurement range, is stored in the nonvolatile memory 113, the second air tank 32 is rapidly exhausted, and procedures for the second air tank 32 are finished (S007 to S009).

Through the above procedures, D/A output signal value groups at respective pressure points in a case of connecting the second air tank 32 (i.e., in a case of the arm circumferential length of 320 mm) are stored in the nonvolatile memory 113. Thus, the basic data stored and held in the nonvolatile memory 113, is configured by these D/A output signal value groups.

The basic data stored in the nonvolatile memory 113 may be the D/A output signal value itself at each pressure point or graphic data obtained by means of the linear interpolation of those pressure points (see FIG. 7). That is, as far as the circumferential length of the measurement site corresponding to the D/A output signal value can be determined based on the pressure value and the D/A output signal value, its data form is not particularly limited.

After the above-described measurement and writing of the basic data are finished, the basic data measuring means 127a sends an instruction to the adjustment jig 30 to change the air tank connected to the sphygmomanometer body 10 (S011), if procedures for all tanks in the adjustment jig 30 have not finished (S010). Namely, from the sphygmomanometer body 10, the basic data measuring means 127a sends information for specifying the other tank of two air tanks, to the adjustment jig 30 through the communication ports 127b and 35. When the information is received, in the adjustment jig 30, the tank switching control circuit 34 sends an operating instruction to the tank switching means 33. As a result, in the adjustment jig 30, the tank connected to the sphygmomanometer body 10 is switched not to the second air tank 32 but to the first air tank 31 having an air volume of 200 cc corresponding to the lower limit of the range of the adaptable upper-arm circumferential length for the adult cuff.

Once the first air tank 31 is connected, the relevant switching information is sent from the tank switching control circuit 34 to the sphygmomanometer body 10 through the communication ports 127b and 35. When the switching information is received, the sphygmomanometer body 10 conducts a measurement of basic data by using the first air tank 31 and writes the data into the nonvolatile memory 113 (S004 to S009) in the same procedures as the second air tank 32.

Thus, in addition to the basic data in the case of connecting the second air tank 32 (namely in the case of the arm circumferential length of 320mm), the basic data in the case of connecting the first air tank 31 (namely, in the case of the arm circumferential length of 240mm) is stored and held in the nonvolatile memory 113.

The basic data may also be the D/A output signal value itself at each pressure point in the same manner as the second air tank 32 or graphic data obtained by means of the linear interpolation of those pressure points (see FIG. 7); wherein its data form is not particularly limited. However, The same data form is preferable between the first air tank 31 and the second air tank 32.

When the measurement of basic data of at least two arm circumferential lengths per one cuff band 20 and storage of the data in the nonvolatile memory 113 are ended, the pre-processing for the cuff band 20 is completed. However, if there are more cuff bands 20 expected to be used, measurement of basic data regarding those cuff bands 20 should be conducted and the obtained basic data is stored in the nonvolatile memory 113 as well. When the measurement and storage of basic data of all cuff bands 20 expected to be used are ended, the pre-processing is completed.

Here, an example is given for a case that the processing for the first air tank is performed after processing for the second air tank. However, an order of the processing is not particularly limited and the reverse order is possible.

### (3-2. Blood pressure measurement procedure)

Next, a procedure for blood pressure measurement using basic data obtained by the pre-processing, and particularly, operation control procedures conducted by the processor 115 of the sphygmomanometer body 10 at the time of the blood pressure measurement will be described in detail.

Here, an example is given for a case that a highest blood pressure value of the subject is 150mmHg, a lowest blood pressure value of the subject is 90mmHg, and a target pressurization value is set to 180mmHg. However, those values are merely specific examples and actual values are not limited thereto.

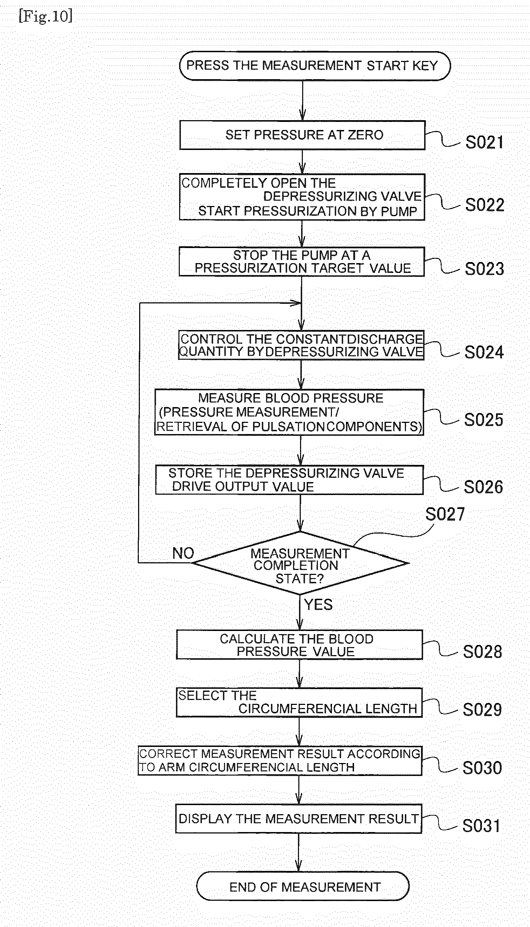

FIG. 10 is a flow chart showing an example of a specific procedure for the method of measuring a blood pressure using the electronic sphygmomanometer according to the present invention.

When the start button 109 is pressed with the cuff band 20 wrapped around a subject's measurement site and the pressurization target value set at 180 mmHg by means of the operation button 108, the processor 115 starts the following operational control.

First, in the processor 115, an operating instruction is sent to the pressure pump drive circuit 111 from the pressurization controller 121, with the electric depressurizing valve 103 closed, after zero-setting (reference matching) of the pressure detected by the pressure sensor 104 is performed (S021), and when the pressure sensor 104 detects that the pressure in the bladder 201 has reached 180 mmHg which is the pressurization target value, the pressurizing controller 121 stops the operation of the pressure pump 102 (S023).

Subsequently, in the processor 115, the depressurizing controller 123 sends an instruction to the electric depressurizing valve drive circuit 112 so that the pressure in the bladder 201 is reduced by the electric depressurizing valve 103. However, at this point, the depressurizing controller 123 controls the depressurizing operation conducted by the electric depressurizing valve 103 by feedback so as to depressurize the inside of the bladder 201 at a specific speed (e.g., 3 mmHg per second) (S024). Specifically, based on the result detected by the pressure sensor 104 and the timing result obtained by the timing means 122, the depressurizing controller 123 determines the drive current value of the electric depressurizing valve 103 in such a way that if a depressurizing speed is faster than 3 mmHg per second which is a depressurizing target speed, the drive current of the electric depressurizing valve 103 is increased; and if depressurizing speed is slower than 3 mmHg per second, the drive current is decreased. Then, the D/A output signal value uniquely corresponding to the selected drive current value is given to the electric depressurizing valve drive circuit 112. Thus, the depressurization speed for depressurizing the inside of the bladder 201 by the electric depressurizing valve 103, is controlled to 3mmHg/second being the target value.

In the above process of the depressurization at a specific depressurizing speed, when the depressurizing speed is stably controlled to a target value (specifically, when a speed variation is within a prescribed allowable range), the blood pressure measuring means 125 of the processor 115 conducts blood pressure measurement to the measurement site wrapped by the cuff band 20 (S025). Specifically, for example, when using the oscillometric method based on the detected pressure results obtained in the process of gradually reducing the pressure added on the measurement site, pulse components that are superposed on the detected pressure data are sorted. This process will be conducted until measurement end conditions are satisfied (S027), and the blood pressure value is then calculated based on the subsequently sorted pulse signals and pressure data (S028). As a result, if, for example, the highest blood pressure of the subject is 150 mmHg and the lowest blood pressure of the subject is 90 mmHg, the depressurizing controller 123 ends the depressurization at a specific depressurizing speed, for example, at the pressure around 70 mmHg, which is lower by a specific amount than the highest blood pressure, and the bladder 201 is exhausted rapidly.

Incidentally, in the processor 115, the circumferential length estimating means 124 performs processing for obtaining the circumferential length of the measurement site, in parallel to the blood pressure measurement by the blood pressure measuring means 125, in the depressurization process. Although it is desirable that these operations be conducted concurrently, they may be conducted subsequently.

To obtain the circumferential length of the measurement site, the circumferential length estimating means 124 first records the D/A output signal values corresponding to each of a plurality of pressure values in the depressurization process, in the RAM of the processor 115 (S026). Specifically, the processing is started for pressurizing the inside of the bladder 201 to 180mmHg, and thereafter obtaining the circumferential length of the measurement site from 160mmHg where the depressurizing speed is stably controlled to a target value. Then, the D/A output signal value corresponding to the drive current value of the electric depressurizing valve 103, is associated with a pressure value corresponding thereto, at a specific interval (for example every time of 20mmHg depressurization) from a point of 160mmHg to a point of 80mmHg for example before the end of the depressurization (for example, in the vicinity of 70mmHg for example), and the obtained D/A output signal value is recorded in the RAM of the processor 115. Thus, the D/A output signal value corresponding to each of the plurality of pressure values in the bladder 201 is stored in the RAM.

When the D/A output signal value is recorded in the RAM, the circumferential length estimating means 124 uses the basic data read from the nonvolatile memory 113 and calculates the circumferential length of the measurement site corresponding to the D/A output signal value (S029). Specifically, one D/A output signal value (hereafter, referred to as a "calculation target signal value") in the data stored in the RAM is focused, and the pressure value corresponding to the calculated target signal value is recognized. Also, from the basic data in the case that the circumferential length is 320 mm which is the upper limit of the range of the adaptable upper-arm circumferential length (i.e., in the case of the second air tank 32), the D/A output signal value corresponding to the pressure value (hereafter, referred to as an "upper limit signal value") is recognized; and from the basic data in the case that the circumferential length is 240 mm which is the lower limit of the range of the adaptable upper-arm circumferential length (i.e., in the case of the first air tank 31), the D/A output signal value corresponding to the pressure value (hereafter, referred to as a "lower limit signal value") is recognized. At this time, if the basic data includes the D/A output signal value itself at each pressure point, the upper limit signal value and the lower limit signal value can be recognized by means of the linear interpolation of each pressure point. After the upper limit signal value and the lower limit signal value are recognized, calculation is conducted to obtain the point between the upper limit signal value and the lower limit signal value where the calculation target signal value is located (i.e., the size (cm) of the arm circumferential length), which is the circumferential length of the measurement site corresponding to the calculation target signal value. The calculation may be done by means of the linear interpolation based on the upper limit signal value and the lower limit signal value (i.e., signal values for different air volumes). However, if the circumferential length of the measurement site corresponding to the calculation target signal value is obtained by use of the specific calculation formula based on each signal value, other well-known calculation methods may be used. The circumferential length estimating means 124 conducts such calculations to obtain the circumferential length of the measurement site for all of the D/A output signal values corresponding to each pressure value recorded in the RAM. After the circumferential length of the measurement site is obtained, the circumferential length estimating means 124 records the result in the RAM of the processor 115. Thus, the circumferential length of the measurement site corresponding to each D/A output signal value (calculation target signal value) recorded in the RAM is stored in the RAM.

Calculation of the circumferential length of the measurement site and recording of the data in the RAM are expected to be conducted every time the circumferential length estimating means 124 records the calculation target signal value in the RAM. However, such recording is not necessarily required to be conducted every time as described above, and calculation and recording of the circumferential length of the measurement site may be performed into the RAM collectively for each calculation target signal value, after end of recording all of a plurality of calculation target signal values into the RAM.

When the circumferential length estimating means 124 performs calculation and recording of the circumferential length of the measurement site into the RAM, there are a plurality of calculation results in the RAM, which are the calculation results of the circumferential lengths of the measurement sites for each of the plurality of pressure values in the depressurization process of the inside of the bladder 201.

Thus, when a plurality of calculation results of the circumferential lengths of a plurality of measurement sites are obtained, the circumferential length estimating means 124 specifies one reference circumferential length form each calculation result (S029). Specifically, the mean value, most frequently-appearing value, or the intermediate value of each calculation result is calculated, and the obtained value is used as one reference circumferential length. Thus, for example, an adverse influence such as a variation that can be generated in each calculation result is excluded, and high precision is achieved in the circumferential length of the measurement site obtained by the circumferential length estimating means 124.

Note that one reference circumferential length is specified by mean calculation, etc., when a plurality of calculation results are obtained by the circumferential length estimating means 124. For example, if the circumferential length estimating means 124 does not obtain the circumferential length of the measurement site at a plurality of pressure values in the process of depressurizing the inside of the bladder 201, but obtains the circumferential length of the measurement site only at one pressure value previously set in the depressurization process, the circumferential length of the measurement site at one pressure value can be used as one reference circumferential length.

After the circumferential length estimating means 124 specifies one reference circumferential length, the measurement result correcting means 126 corrects the blood pressure measurement result obtained by the blood pressure measuring means 125 based on the specified reference circumferential length (S030). For example, when the Ragan & Bordley correction table (see FIG. 4) is applied, the correction value corresponding to the reference circumferential length is determined based on the correction table, and the measurement result is corrected by adding the correction value to the blood pressure measurement result or subtracting the correction value from the blood pressure measurement result. More specifically, if the reference circumferential length is 240 mm, based on the Ragan & Bordley correction table, 5 mmHg is added to the measured highest blood pressure value 150 mmHg, and 5 mmHg is subtracted from the measured lowest blood pressure value 90 mmHg.

After the measurement result correcting means 126 corrects the blood pressure measurement results, the processor 115 of the sphygmomanometer body 10 outputs and displays the correction results (i.e., corrected highest blood pressure value and lowest blood pressure value) on the LCD 110 provided on the sphygmomanometer body 10 (S031). When the LCD 110 displays the corrected highest blood pressure value and lowest blood pressure value, the processor 115 finishes the blood pressure measurement of the subject.

### <4. Effects in this embodiment>

Next, effects of this embodiment will be described.
(i) In this embodiment, the circumferential length of the measurement site wrapped by the cuff band 20 is obtained, using the drive current of the electric depressurizing valve 103 regulated by the electric depressurizing valve drive circuit 112 at the time of depressurizing the inside of the bladder 201, and the basic data that has been previously set in the nonvolatile memory 113. Herein, the drive current of the electric depressurizing valve 103 corresponds to a specific example of the driving energy of the electric depressurizing valve 103 and is regulated to depressurize the inside of the bladder 201 at a specific speed (slow leak speed). The depressurization of the inside of the bladder 201 at a specific speed, is required for non-invasive blood pressure measurement. Namely, the function of regulating the drive current of the electric depressurizing valve 103 to depressurize the inside of the bladder 201 at a specific speed is a function that should be originally provided in the electronic sphygmomanometer. Therefore, if the circumferential length of the measurement site is obtained using the drive current of the electric depressurizing valve 103, the circumferential length can be obtained by using the function originally provided in the electronic sphygmomanometer (slow leak speed control function) without additionally providing a special detecting function for the cuff band 20 or the pressure pump 102 that pressurizes the inside of the bladder 201 of the cuff band 20. Accordingly, when compared with a case that a special detecting function is required, there is less risk of occurrence of a failure and cost reduction is easily achieved.
   Incidentally, the circumferential length of the measurement site is expected to be obtained based on the information obtained in the process of pressurizing the inside of the bladder 201 (e.g., the discharge flow rate of the air discharged into the bladder 201) as shown in the conventional technology disclosed in patent literature 1. However, in this case, difference is generated in the pressurization process depending on a wrapping state of the cuff band 20 (for example, depending on a state whether the cuff band 20 is tightly or loosely wrapped around the measurement site), and there is a possibility that the circumferential length estimation result is affected thereby.
   Meanwhile, as described in this embodiment, if the circumferential length of the measurement site is obtained by use of the drive current of the electric depressurizing valve 103, the information obtained in the process of depressurizing the inside of the bladder 201 from a state of being pressurized to a prescribed pressure, with the cuff band 20 air-tightly adhered to the circumference of the measurement site, is based. Therefore, the influence of the wrapping state of the cuff band 20 can be avoided. Accordingly, by using the drive current of the electric depressurizing valve 103, the circumferential length of the measurement site can be precisely obtained, compared with a case that the information obtained in the pressurization process of the inside of the bladder 201, is based.
   Furthermore, if a circumferential length of the measurement site is obtained in the process of pressurizing the inside of the bladder 201, for example, it can be probably obtained based on a pressurizing time up to a prescribed pressure. However, in this case, there is a variation in the time required for the pressurization up to a specific pressure due to operational fluctuation factors of the pressure pump 102 or the pressure pump drive circuit 111 (e.g., uneven pressurizing capabilities or changes of drive voltage), and there is a possibility that such a fluctuation may affect the estimation result of the circumferential length.
   Meanwhile as described in this embodiment, by using the drive current of the electric depressurizing valve 103, the circumferential length of the measurement site can be obtained in the state that the discharge flow rate of the air discharged from the bladder 201 is controlled to perform depressurization at a specific speed, namely the state that operational fluctuation factors of the electric depressurizing valve 103 are eliminated by the regulation of the drive current. Therefore, by using the drive current of the electric depressurizing valve 103, the circumferential length of the measurement site can be precisely obtained, compared with a case that the circumferential length of the measurement site is obtained in the pressurization process of the inside of the bladder 201. Namely, an estimation error is not generated in the arm circumferential length, due to the difference of the performance or the pressurizing capabilities of the pressure pump 102.
(ii) In this embodiment, the blood pressure measurement result of the measurement site is corrected according to the circumferential length of the measurement site obtained by using the drive current of the electric depressurizing valve 103. Accordingly, even when the cuff bands 20 having a plurality of sizes are prepared responding to various subjects and one of those cuff bands is selected and used, the measurement error of the blood pressure value resulting from incompatibility between the circumferential length of the measurement site and the width of the cuff band 20 is eliminated, and a precise blood pressure measurement result can be obtained. Namely, even when there are a variety of cuff bands 20 used, the blood pressure measurement result can be precisely corrected.
(iii) In this embodiment, out of the drive energy amount of the electric depressurizing valve 103, the drive current value corresponding to a specific example is used to obtain the circumferential length of the measurement site wrapped by the cuff band 20. The drive current value of the electric depressurizing valve 103 has a unique correlation with the pressure in the bladder 201 and the air volume as shown in FIG. 6. Therefore, as described in this embodiment, if the circumferential length of the measurement site is obtained using the drive current of the electric depressurizing valve 103, the circumferential length of the measurement site can be uniquely derived (reliably and precisely).
   Furthermore, by using the drive current of the electric depressurizing valve 103, a stable drive current can flow as the drive current without being affected by fluctuation of power-supply voltage or temperature, provided that the electric depressurizing valve drive circuit 112 includes a constant current circuit. For example, even if there is a variation in supply voltage to the constant current circuit, by controlling the electric depressurizing valve 103 within the operation range of the supply voltage, the electric depressurizing valve 103 is not affected by power-supply voltage variation. Therefore, the adverse effect of the variation in power-supply voltage or temperature., can be prevented when the circumferential length of the measurement site is obtained. In this regard, it is preferable to use the drive current value as the drive energy amount of the electric depressurizing valve 103. Namely, the adverse effects of the variation in power-supply voltage at the measurement of the arm circumferential length, can be prevented.
(iv) In this embodiment, to obtain the circumferential length of the measurement site, the D/A output signal value, which is an example of the information equivalent to the drive current value of the electric depressurizing valve 103 is used. If the circumferential length of the measurement site is obtained from the D/A output signal value, even when the circumferential length is obtained using the drive current of the electric depressurizing valve 103, the drive current value regulated by the electric depressurizing valve drive circuit 112 is not required to be detected. Namely, even if the circumferential length estimating means 124 of the processor 115 does not monitor the drive current regulated by the electric depressurizing valve drive circuit 112, the circumferential length of the measurement site can be obtained only by recognizing the D/A output signal value sent to the electric depressurizing valve drive circuit 112 from the depressurizing controller 123 of the processor 115.
   Even in that case, since the D/A output signal value uniquely corresponds to the drive current value, the same result as obtained in the case of detecting the drive current value can be obtained.
(v) In this embodiment, when obtaining the circumferential length of the measurement site, previously set basic data is used in addition to the drive current value of the electric depressurizing valve 103. The basic data defines the correlation between the pressure in the bladder 201 and the drive current of the electric depressurizing valve 103 in the case of a specific circumferential length. When the pressure value in the bladder 201 and the drive current value of the electric depressurizing valve 103 or the D/A output signal value, which is an example of the information equivalent to the drive current value, are given, the circumferential length of the measurement site corresponding to those values can be determined. Thus, the basic data may be any data that can sufficiently determine the circumferential length of the measurement site, namely, the data capable of sufficiently deriving the circumferential length of the measurement site from the pressure value in the bladder 201 and the drive current value of the electric depressurizing valve 103 or the D/A output signal value, can be used.
   In other words, although there is the correlation of the circumferential length of the measurement site, the air volume of the bladder 201, the pressure in the bladder 201 at the time of the depressurization of the inside of the bladder 201, and the drive current of the electric depressurizing valve 103, the typical correlation may be defined by the basic data. Therefore, even when the basic data is previously set, its extremely large data amount cannot be suppressed.
   Furthermore, as described in this embodiment, if the basic data is prepared in advance and the circumferential length of the measurement site is obtained by referring to the prepared basic data, the calculation processing, etc., for obtaining the circumferential length is prevented from being more complicated than a case of not using the basic data. Namely, owing to the presence of the basic data, excessively high processing load can be suppressed for obtaining the circumferential length of the measurement site, and speedy processing and a simplified configuration required for the processing can be expected.
   Furthermore, when the circumferential length of the measurement site is obtained by referring to the prepared basic data, individual basic data can be prepared for each electronic sphygmomanometer. Namely, the basic data responding to uneven characteristics (individual difference) of the electric depressurizing valve 103 can be prepared beforehand. Therefore, the circumferential length of the measurement site can be obtained, irrespective of such an uneven characteristic.
   When the electric depressurizing valve 103 does not have individual characteristic unevenness, or if the characteristic unevenness is within the range that does not practically affect the execution of the arm circumferential length estimating means of the present invention, the typical value corresponding to the electric depressurizing valve 103 can be used as a fixed value, and the processes of measuring the basic data and storage of the basic data in the nonvolatile memory 113 are not required.
   Meanwhile, in order to eliminate the individual characteristic unevenness of the electric depressurizing valve 103 or to limit the characteristic unevenness within the range that does not practically affect the execution of the arm circumferential length estimating means of the present invention, production costs is increased accordingly, which is not preferable in terms of realizing a product.
   The process of measuring basic data and storage of the basic data in the nonvolatile memory 113 according to the present invention can contribute to solving the problems and reducing costs.
(vi) In this embodiment, in order to obtain the circumferential length of the measurement site, the circumferential length of the measurement site is obtained for a plurality of pressure values in the process of depressurizing the inside of the bladder 201, and one reference circumferential length is specified from each of the circumferential lengths on the plurality of pressure values. Thus, for example, the adverse effects of unevenness in the circumferential length calculation results for the plurality of pressure values, can be eliminated, and more precision is achieved in the circumferential length of the measurement site obtained by the circumferential length estimating means 124 of the processor 115. Namely, by correcting the blood pressure measurement result based on the specified one reference circumferential length, the blood pressure measurement result can be corrected precisely, without being influenced by the adverse influence of the measurement variation, even if the measurement variation is generated in each calculation result of the circumferential lengths in a plurality of pressure values.
(vii) In this embodiment, the nonvolatile memory 113 stores the basic data. Furthermore, while the adjustment jig 30 is connected to the electronic sphygmomanometer, the adjustment jig 30 performs processing of performing measurement for specifying the basic data and writing of the basic data obtained by the measurement, into the nonvolatile memory 113, using the sphygmomanometer. Therefore, by connecting the adjustment jig 30, prior measurement of the basic data can be performed for each sphygmomanometer, and the result thereof can be written into the nonvolatile memory 113 in each sphygmomanometer. Namely, the basic data specific to each electronic sphygmomanometer can be stored in the nonvolatile memory 113 of each electronic sphygmomanometer. Thus, regarding the basic data referenced by the circumferential length estimating means 124 of the processor 115 when obtaining the circumferential length of the measurement site, individual difference in each electronic sphygmomanometer can be eliminated through the individual prior measurement using the adjustment jig 30. In other words, the correlation including characteristic unevenness of the electric depressurizing valve 103 in each electronic sphygmomanometer is stored as basic data in the nonvolatile memory 113 of each electronic sphygmomanometer. Therefore, the circumferential length of the measurement site can be obtained without being influenced by the characteristic unevenness.

### <5. Modified example>

In this embodiment, explanation is given for suitable specific examples of the electronic sphygmomanometer, the method of estimating a circumferential length of the measurement site, and the method of measuring a blood pressure according to the present invention. However, the present invention is not limited thereto.

For example, in this embodiment, an example is given for a case that the drive current value being a specific example of the drive energy amount of the electric depressurizing valve 103 is used for obtaining the circumferential length of the measurement site. However, the present invention is not limited thereto, and other drive energy amount can be used.

A power amount which is a work amount of a current per unit time can be considered as other drive energy amount.

The power amount is particularly preferable if being applied to a case that the control to the electric depressurizing valve 103 is performed by PWM (Pulse Width Modulation) control.

Furthermore, the drive voltage value of the electric depressurizing valve 103 can also be used as further other drive energy amount. Thus, the drive energy amount of the electric depressurizing valve 103 is not limited to the drive current value, and may be suitably obtained according to a control mode of the electric depressurizing valve 103. However, as described in this embodiment, in terms of eliminating the adverse effects of power-supply voltage variation by means of the constant current circuit, the drive current value is preferably used as the drive energy amount.

Furthermore, in this embodiment, an example is given for a case that the circumferential length of the measurement site is obtained, and thereafter by using the result thereof, the measurement result of the blood pressure value is corrected. However, the present invention is not limited thereto. Namely, the circumferential length of the measurement site obtained by using the drive energy amount of the electric depressurizing valve 103 is not sued for correcting the measurement result of the blood pressure value, but can be used for other purpose together with the user for correcting the measurement result of the blood pressure value. For example as other purpose of use, it can be considered that the circumferential length of the measurement site is stored and held in a database form, which is then used for managing the circumferential length of the measurement site of the subject.

Furthermore, in this embodiment, explanation is given for the size of the cuff band 20, the air volume in the bladder 201, the pressure in the bladder 201, the speed for depressurizing the inside of the bladder 201, and the blood pressure value of the subject, etc., with specific numerical values given therefore. However, of course these numerical values are merely specific examples and can be suitably set or changed as needed.

Furthermore, in this embodiment, explanation is given for a case that the "adult cuff" having a highest distribution ratio of the arm circumferential length is given as an example. However, the present invention can completely similarly applied to other cuff band (see FIG. 11) .

Moreover, according to the present invention, one cuff band can respond to a wide range of arm circumferential length. Therefore, it can be considered that one cuff band can respond to the wide range of the arm circumferential length such as a range of 240mm to 420mm, by setting a only a cuff cloth of the adult cuff to be long, which is the adult cuff having an adaptable range length of 240mm to 320mm, so that an area of a large cuff having a length of 320mm to 420mm can be wrapped by one cuff band.

Furthermore, in this embodiment, explanation is given for a case that the adjustment jig 30 automatically conducts basic data measurement in the pre-processing. However, the present invention is not limited thereto, and the adjustment operator may manually conduct the operation as described below. Namely, when manually conducting the basic data measurement, switch of an operation mode to a regulation mode in the sphygmomanometer body 10 (start of a regulation mode function 127) is enabled by a manual switching operation in the sphygmomanometer body 10.

Furthermore, a tank value to be connected to outside by LCD 110 of the sphygmomanometer body 10 is made displayable. Moreover, at least two air tanks having mutually different air volumes are prepared by an adjustment operator, and selective connection and exchange of the air tank to/with a connector 101 of the sphygmomanometer body 10 is performed manually by the adjustment operator. Then, a start instruction of measuring the basic data is performed through the start button 109 of the sphygmomanometer body 10 in a state of connecting the air tank. Thus, only by preparing the air tank having a specific volume, the measurement and storage (setup) of the basic data can be performed. Namely, the measurement and storage of the basic data can be performed without a dedicated adjustment jig 30, which is effective for a case of re-adjustment, etc., of the basic data, particularly when exchanging the electric depressurizing valve 103. Moreover, since the communication port 127b, etc., for connecting the adjustment jig 30 is not required, which contributes to a cost reduction of the sphygmomanometer body 10.

Furthermore, when the basic data measurement is automatically conducted using the adjustment jig 30, in this embodiment, the sphygmomanometer body 10 and the adjustment jig 30 include the communication ports 127b and 35, to conduct bidirectional communication. However, the present invention is not limited thereto. For example, when using a publicly-known technique (see patent publication No.4467263 for example) of performing a specific control by extracting a digital code signal from a variation in power supply voltage, the measurement and storage of the basic data can be performed, even if the communication between the sphygmomanometer body 10 and the adjustment jig 30 is unidirectional. Specifically, the power-supply voltage of the sphygmomanometer body 10 is supplied from the adjustment jig 30, and information for switching the operation mode of the sphygmomanometer body 10 is transmitted from the adjustment jig 30 to the sphygmomanometer body 10 using the above-mentioned well-known technique. Then, only output of an air tank selection signal is performed to the adjustment jig 30 from the sphygmomanometer body 10. With this structure, bidirectional communication ports 127b, 35 are not required between the sphygmomanometer body 10 and the adjustment jig 30, thus contributing to the cost reduction.

As stated above, the present invention is not limited to the above-mentioned contents of this embodiment and can be suitably modified without departing from the scope of the invention.

### {Reference Signs List}

10...sphygmomanometer body, 20...cuff band, 30...adjustment jig, 102...pressure pump, 103...electric depressurizing valve, 111...pressure pump drive circuit, 112...electric depressurizing valve drive circuit, 113...nonvolatile memory, 115...processor, 121...pressurizing controller, 122...timing means, 123...depressurizing controller, 124...circumferential length estimating means, 125...blood pressure measuring

## Claims

1. An electronic sphygmomanometer (10) comprising:
a cuff band (20) wrapped around a measurement site;
a fluid bag (201) included in the cuff band;
pressurizing means (102) for pressurizing an inside of the fluid bag by supplying a fluid into the fluid bag;
an electric depressurizing valve (103) for reducing a pressure in the fluid bag by discharging the fluid from the fluid bag pressurized to a specific pressure;
an electric depressurizing valve drive circuit (112) for regulating a drive energy amount of the electric depressurizing valve for controlling a discharge flow rate of the fluid discharged by the electric depressurizing valve, to thereby reduce a pressure in the fluid bag at a constant depressurizing speed; and
circumferential length estimating means (124) for obtaining a circumferential length of the measurement site by using the drive energy amount regulated by the electric depressurizing valve drive circuit and previously set basic data, wherein
the basic data is configured to define a correlation between the pressure in the fluid bag and the drive energy amount of the electric depressurizing valve at a prescribed circumferential length, so that the circumferential length of the measurement site corresponding to the drive energy amount or information corresponding thereto is specified when the pressure value in the fluid bag and the drive energy amount or the information corresponding thereto are given.

2. The electronic sphygmomanometer according to claim 1, further comprising:
blood pressure measuring means (125) for conducting blood pressure measurement to the measurement site in a process of depressurizing the inside of the fluid bag; and
measurement result correcting means (126) for correcting a blood pressure measurement result obtained by the blood pressure measuring means based on the circumferential length of the measurement site obtained by the circumferential length estimating means.

3. The electronic sphygmomanometer according to claim 1 or 2, wherein the drive energy amount is a drive current value of the electric depressurizing valve.

4. The electronic sphygmomanometer according to claim 3, further comprising:
a depressurizing controller (123) for giving a regulating instruction to the electric depressurizing valve drive circuit, the regulating instruction being an instruction of regulating the drive current value by sending a control signal showing information equivalent to the drive current value of the electric depressurizing valve, wherein
the circumferential length estimating means obtains a circumferential length of the measurement site by using the control signal sent to the electric depressurizing valve drive circuit from the depressurizing controller.

5. The electronic sphygmomanometer according to any one of claims 1 to 4, wherein
the circumferential length estimating means obtains circumferential lengths of measurement sites at a plurality of pressure values in a process of depressurizing an inside of the fluid bag, and specifies one reference circumferential length from the circumferential lengths at the plurality of pressure values.

6. The electronic sphygmomanometer according to claim 5, wherein the one reference circumferential length is a mean value of the obtained circumferential lengths, a most frequently-appearing value thereof, or an intermediate value located between a maximum value and a minimum value thereof.

7. The electronic sphygmomanometer according to any one of claims 1 to 6, further comprising:
data storage means (113) for storing the basic data; and
connecting means (127) for connecting an adjustment jig for conducting measurement for specifying the basic data and writing the basic data obtained by the measurement into the data storage means.

8. A method of measuring blood pressure comprising:
pressurizing an inside of a fluid bag by supplying a fluid into the fluid bag while a cuff band internally including the fluid bag is wrapped around a measurement site;
reducing a pressure in the fluid bag by discharging the fluid from the fluid bag pressurized to a specific pressure by using an electric depressurizing valve, and regulating a drive energy amount of the electric depressurizing valve to control a discharge flow rate of the fluid discharged by the electric depressurizing valve so that a depressurizing speed becomes a constant speed when the pressure of the inside of the fluid bag is reduced; and
obtaining a circumferential length of the measurement site by using the regulated drive energy amount of the electric depressurizing valve and previously set basic data, wherein
the basic data is configured to define a correlation between the pressure in the fluid bag and the drive energy amount of the electric depressurizing valve at a prescribed circumferential length, so that the circumferential length of the measurement site corresponding to the drive energy amount or information corresponding thereto is specified when the pressure value in the fluid bag and the drive energy amount or the information corresponding thereto are given.

9. The method of measuring blood pressure according to claim 8, further comprising:
conducting blood pressure measurement to the measurement site in a process of reducing the pressure in the fluid bag; and
correcting a result of a blood pressure measurement applied to the measurement site based on the circumferential length of the measurement site obtained by using the drive energy amount of the electric depressurizing valve.

## Patentansprüche

1. Elektronisches Sphygmomanometer (10), umfassend:
ein Manschettenband (20), das um eine Messstelle herum gewickelt ist;
eine Fluidtasche (201), die in dem Manschettenband enthalten ist;
Druckbeaufschlagungsmittel (102) zum Beaufschlagen eines Inneren der Fluidtasche mit Druck durch Zuführen eines Fluids in die Fluidtasche;
ein elektrisches Drucksenkungsventil (103) zum Reduzieren eines Drucks in der Fluidtasche durch Auslassen des Fluids von der Fluidtasche, die auf einen spezifischen Druck druckbeaufschlagt ist;
eine elektrische Drucksenkungsventiltreibschaltung (112) zum Regeln einer Treibenergiemenge des elektrischen Drucksenkungsventils zum Steuern einer Auslassflussrate des Fluids, das durch das elektrische Drucksenkungsventil ausgelassen wird, um hierdurch einen Druck in der Fluidtasche bei einer konstanten Drucksenkungsgeschwindigkeit zu reduzieren; und
Umfangslängenabschätzungsmittel (124) zum Erhalten einer Umfangslänge der Messstelle unter Verwendung der Treibenergiemenge, geregelt durch die elektrische Drucksenkungsventiltreibschaltung, und zuvor eingestellte Basisdaten, wobei
die Basisdaten dazu konfiguriert sind, eine Korrelation zu definieren zwischen dem Druck in der Fluidtasche und der Treibenergiemenge des elektrischen Drucksenkungsventils bei einer vorgeschriebenen Umfangslänge, so dass die Umfangslänge der Messstelle entsprechend der Treibenergiemenge oder einer Information entsprechend hierzu spezifiziert ist, wenn der Druckwert in der Fluidtasche und die Treibenergiemenge oder die Information entsprechend hierzu gegeben sind.

2. Elektronisches Sphygmomanometer nach Anspruch 1, ferner umfassend:
Blutdruckmessmittel (125) zum Durchführen einer Blutdruckmessung an der Messstelle in einem Prozess des Drucksenkens des Inneren der Fluidtasche; und
Messergebniskorrekturmittel (126) zum Korrigieren eines Blutdruckmessergebnisses, das durch die Blutdruckmessmittel erhalten ist, basierend auf der Umfangslänge der Messstelle, die durch die Umfangslängenabschätzmittel erhalten ist.

3. Elektronisches Sphygmomanometer nach Anspruch 1 oder 2, wobei die Treibenergiemenge ein Treibstromwert des elektrischen Drucksenkungsventils ist.

4. Elektronisches Sphygmomanometer nach Anspruch 3, ferner umfassend:
einen Drucksenkungscontroller (123) zum Ausgeben einer Regelinstruktion an die elektrische Drucksenkungsventiltreibschaltung, wobei die Regelinstruktion eine Instruktion zum Regeln des Treibstromwerts durch Senden eines Steuersignals ist, das eine Information äquivalent zu dem Treibstromwert des elektrischen Drucksenkungsventils zeigt, wobei
die Umfangslängenabschätzmittel eine Umfangslänge der Messstelle unter Verwendung des Steuersignals erhalten, das zu der elektrischen Drucksenkungsventiltreibschaltung von dem Drucksenkungscontroller gesendet wird.

5. Elektronisches Sphygmomanometer nach einem der Ansprüche 1 bis 4, wobei die Umfangslängenabschätzmittel Umfangslängen von Messstellen bei einer Mehrzahl von Druckwerten in einem Prozess des Drucksenkens eines Inneren der Fluidtasche erhalten und eine Referenzumfangslänge von den Umfangslängen bei der Mehrzahl von Druckwerten spezifizieren.

6. Elektronisches Sphygmomanometer nach Anspruch 5, wobei die eine Referenzumfangslänge ein Mittelwert der erhaltenen Umfangslängen ist, ein am häufigsten auftretender Wert davon, oder ein Zwischenwert, der zwischen einem maximalen Wert und einem minimalen Wert davon lokalisiert ist.

7. Elektronisches Sphygmomanometer nach einem der Ansprüche 1 bis 6, ferner umfassend:
Datenspeichermittel (113) zum Speichern der Basisdaten; und
Verbindungsmittel (127) zum Verbinden einer Anpassungsschablone zum Durchführen einer Messung zum Spezifizieren der Basisdaten und Schreiben der Basisdaten, die durch die Messung erhalten sind, in die Datenspeichermittel.

8. Verfahren zur Blutdruckmessung, umfassend:
Beaufschlagen eines Inneren einer Fluidtasche mit Druck durch Zuführen eines Fluids in die Fluidtasche, während ein Manschettenband, das die Fluidtasche intern enthält, um eine Messstelle herum gewickelt ist;
Reduzieren eines Drucks in der Fluidtasche durch Auslassen des Fluids von der Fluidtasche, die auf einen spezifischen Druck unter Druck gesetzt ist, unter Verwendung eines elektrische Drucksenkungsventils, und Regeln einer Treibenergiemenge des elektrischen Drucksenkungsventils zum Steuern einer Auslassflussrate des Fluids, das durch das elektrische Drucksenkungsventil ausgelassen wird, so dass eine Drucksenkungsgeschwindigkeit eine konstante Geschwindigkeit wird, wenn der Druck des Inneren der Fluidtasche reduziert wird; und
Erhalten einer Umfangslänge der Messstelle unter Verwendung der geregelten Treibenergiemenge des elektrischen Durcksenkungsventils und zuvor eingestellter Basisdaten, wobei
die Basisdaten dazu konfiguriert sind, eine Korrelation zu definieren zwischen dem Druck in der Fluidtasche und der Treibenergiemenge des elektrischen Drucksenkungsventils bei einer vorgeschriebenen Umfangslänge, so dass die Umfangslänge der Messstelle entsprechend der Treibenergiemenge oder einer Information entsprechend hierzu spezifiziert ist, wenn der Druckwert in der Fluidtasche und die Treibenergiemenge oder die Information entsprechend hierzu gegeben sind.

9. Verfahren zur Blutdruckmessung nach Anspruch 8, ferner umfassend:
Durchführen einer Blutdruckmessung an der Messstelle in einem Prozess des Reduzierens des Drucks in der Fluidtasche; und
Korrigieren eines Ergebnisses einer Blutdruckmessung, die bei der Messstelle angewandt wurde, basierend auf der Umfangslänge der Messstelle, die unter Verwendung der Treibenergiemenge des elektrischen Drucksenkungsventils erhalten wurde.

## Revendications

1. Tensiomètre artériel électronique (10) comprenant :
un brassard (20) enroulé autour d'un site de mesure;
une poche de fluide (201) incluse dans le brassard;
des moyens de pressurisation (102) pour pressuriser l'intérieur de la poche de fluide en y introduisant un fluide ;
une valve électrique de dépressurisation (103) pour réduire la pression dans la poche de fluide en déchargeant le fluide de la poche de fluide pressurisé à une pression spécifique;
un circuit de commande de soupape de dépressurisation électrique (112) pour réguler une quantité d'énergie de commande de la soupape de dépressurisation électrique afin de contrôler un débit de décharge du fluide déchargé par la soupape de dépressurisation électrique, pour réduire ainsi une pression dans la poche de fluide à une vitesse de dépressurisation constante; et
un moyen d'estimation de la longueur circonférentielle (124) pour obtenir une longueur circonférentielle du site de mesure en utilisant la quantité d'énergie motrice régulée par la soupape de dépressurisation électrique et des données de base préalablement définies, dans lequel
les données de base sont configurées pour définir une corrélation entre la pression dans la poche de fluide et la quantité d'énergie d'entraînement de la soupape de dépressurisation électrique à une longueur circonférentielle prescrite, de sorte que la longueur circonférentielle du site de mesure correspondant à la quantité d'énergie d'entraînement ou aux informations qui y correspondent est spécifiée lorsque la valeur de la pression dans la poche de fluide et la quantité d'énergie d'entraînement ou les informations qui y correspondent sont données.

2. Tensiomètre artériel électronique selon la revendication 1, comprenant en outre:
des moyens de mesure de la pression sanguine (125) permettant d'effectuer la mesure de la pression sanguine au site de mesure dans un processus de dépressurisation de l'intérieur de la poche de fluide; et
des moyens de correction des résultats de mesure (126) pour corriger un résultat de mesure de la pression artérielle obtenu par le moyen de mesure de la pression artérielle sur la base de la longueur circonférentielle du site de mesure obtenue par le moyen d'estimation de la longueur circonférentielle.

3. Tensiomètre artériel électronique selon les revendications 1 ou 2, dans lequel la quantité d'énergie motrice est une valeur de courant moteur de la soupape de dépressurisation électrique.

4. Tensiomètre artériel électronique selon la revendication 3, comprenant en outre:
un contrôleur de dépressurisation (123) pour donner une instruction de régulation au circuit de commande de la soupape de dépressurisation électrique, l'instruction de régulation étant une instruction de régulation de la valeur du courant de commande en envoyant un signal de commande montrant des informations équivalentes à la valeur du courant de commande de la soupape de dépressurisation électrique, dans lequel
le moyen d'estimation de la longueur circonférentielle obtient une longueur circonférentielle du site de mesure en utilisant le signal de commande envoyé au circuit de commande de la soupape de dépressurisation électrique par le contrôleur de dépressurisation.

5. Tensiomètre artériel électronique selon l'une des revendications 1 à 4, dans lequel
le moyen d'estimation de la longueur circonférentielle obtient les longueurs circonférentielles des sites de mesure à une pluralité de valeurs de pression dans un processus de dépressurisation de l'intérieur de la poche de fluide, et spécifie une longueur circonférentielle de référence à partir des longueurs circonférentielles à la pluralité de valeurs de pression.

6. Tensiomètre artériel électronique selon la revendication 5, dans lequel la longueur circonférentielle de référence est une valeur moyenne des longueurs circonférentielles obtenues, une valeur de celles-ci apparaissant le plus fréquemment, ou une valeur intermédiaire située entre une valeur maximale et une valeur minimale de celles-ci.

7. Tensiomètre artériel électronique selon l'une des revendications 1 à 6, comprenant en outre:
des moyens de stockage des données (113) pour stocker les données de base; et
des moyens de raccordement (127) pour le raccordement d'un gabarit de réglage pour la réalisation de la mesure pour la spécification des données de base et l'écriture des données de base obtenues par la mesure dans les moyens de stockage des données.

8. Procédé de mesure de la pression artérielle comprenant:
la mise sous pression de l'intérieur d'une poche de fluide en fournissant un fluide dans la poche de fluide tandis qu'une bande de manchette incluant la poche de fluide à l'intérieur est enroulée autour d'un site de mesure;
la réduction de la pression dans la poche de fluide en déchargeant le fluide de la poche de fluide pressurisée à une pression spécifique en utilisant une soupape de dépressurisation électrique, et la régulation d'une quantité d'énergie d'entraînement de la soupape de dépressurisation électrique pour contrôler un débit de décharge du fluide déchargé par la soupape de dépressurisation électrique de sorte qu'une vitesse de dépressurisation devienne une vitesse constante lorsque la pression de l'intérieur de la poche de fluide est réduite; et
l'obtention d'une longueur circonférentielle du site de mesure en utilisant la quantité d'énergie d'entraînement régulée de la soupape de dépressurisation électrique et des données de base préalablement définies, dans lequel
les données de base sont configurées pour définir une corrélation entre la pression dans la poche de fluide et la quantité d'énergie d'entraînement de la soupape de dépressurisation électrique à une longueur circonférentielle prescrite, de sorte que la longueur circonférentielle du site de mesure correspondant à la quantité d'énergie d'entraînement ou aux informations qui y correspondent est spécifiée lorsque la valeur de la pression dans la poche de fluide et la quantité d'énergie d'entraînement ou les informations qui y correspondent sont données.

9. Procédé de mesure de la pression artérielle selon la revendication 8, comprenant en outre:
la mesure de la pression artérielle sur le lieu de mesure dans le cadre d'un processus de réduction de la pression dans la poche de fluide; et
la correction d'un résultat d'une mesure de la pression sanguine appliquée au site de mesure en fonction de la longueur circonférentielle du site de mesure obtenue en utilisant la quantité d'énergie motrice de la soupape de dépressurisation électrique.
